# EUROPEAN PATENT APPLICATION

(11) **EP 4 778 588 A2**
(43) Date of publication of application: **22.07.2026**
(21) Application number: 26183648.0
(22) Date of filing: 26.09.2023
(51) Int. Cl.: A61P 35/00

(54) **ENZYMATICALLY CLEAVABLE LINKER AND LIGAND-ERIBULIN CONJUGATE CONTAINING SAME**

(30) Priority: 20.10.2022 CN 202211287874; 12.07.2023 CN 202310853999
(62) Divisional of application: 23878927.5
(71) Applicant: INNOLAKE BIOPHARMA (HANGZHOU) CO., LTD, Zhejiang Province 310018 (CN)
(72) Inventor: SHANG, Yushuan, Hangzhou City, Zhejiang Province, 310018 (CN); QIU, Junzhuan, Hangzhou City, Zhejiang Province, 310018 (CN); WANG, Zhensheng, Hangzhou City, Zhejiang Province, 310018 (CN); XIA, Mingde, Hangzhou City, Zhejiang Province, 310018 (CN); CHEN, Rulei, Hangzhou City, Zhejiang Province, 310018 (CN)
(74) Representative: Abel & Imray LLP

(57) **Abstract**

The present disclosure relates to the technical field of biomedicines, in particular, relates to an enzymatically cleavable linker and a ligand-eribulin conjugate containing same, and more specifically, relates to the use of VAGGFG as an enzymatically cleavable linker.

## Description

### CROSS-REFERENCE TO RELATED APPLICATIONS

This application claims priority to Patent Application No. CN202211287874.0, filed on October 20, 2022, and entitled "ENZYMATICALLY CLEAVABLE LINKER AND LIGAND-ERIBULIN CONJUGATE CONTAINING SAME", and Patent Application No. CN202310853999.3, filed on July 12, 2023, which are incorporated herein by reference in their entirety.

### TECHNICAL FIELD

The present disclosure relates to the field of biomedicine technology, and specifically, to an enzymatically cleavable linker and a ligand-Eribulin conjugate containing the same.

### BACKGROUND

Antibody-Drug Conjugates (ADCs) are produced by conjugating biologically active small-molecule drugs to monoclonal antibodies (monoclonal antibodies) via linkers. Currently, most ADCs are produced by conjugating antibodies targeting tumor antigens to highly efficient cytotoxic small-molecule chemical drugs via linkers. This allows for the targeted delivery of small-molecule drugs to tumor cells, leveraging the specific binding of antibodies to their target antigens to achieve tumor cell destruction. Additionally, proteins and polypeptides with specific binding properties similar to those of antibodies, such as specific ligands, antigens, and polypeptides, are also conjugated with linker toxins.

Linkers are a critical factor in determining drug stability and therapeutic windows. First, linkers must be stable to ensure the integrity of ADCs during blood circulation before they reach tumor cells. This stability helps prevent premature toxin release, which can result in off-target toxicity and impact the therapeutic window of the ADC drug. Once the ADC enters the target cells, the linker must enable effective toxin release to achieve its cytotoxic effects. Polypeptide linkers are a common type of cleavable linkers. As of September 2022, over 10 ADC drugs have been approved for marketing. Most of these drugs conjugate toxins to the cysteine residues of monoclonal antibodies via polypeptide linkers. These polypeptides can be cleaved by cathepsins within tumor cells, releasing the toxins and thereby exerting antitumor effects.

Currently, valine-citrulline (VC) is the most commonly used in clinical research. The marketed drug Adcetris, for example, employs this linker to conjugate MMAE to the antibody. Other ADCs use linkers such as valine-alanine (VA) and glycine-glycine-phenylalanine-glycine (GGFG), as seen in drugs like SGN-CD33A, Loncastuximab tesirine, and DS-8201a.

As a toxin of antibody conjugates, Eribulin has a high molecular weight, a complex structure, and good hydrophilicity. The high molecular weight necessitates the selection of linkers with appropriate lengths. The complex structure may affect the efficiency of toxin release due to differences in steric hindrance during enzymatic cleavage. Meanwhile, its hydrophilicity makes it well-suited for preparing conjugates with a high drug-to-antibody ratio. Polyethylene glycol (PEG), a commonly used linker component, offers good hydrophilicity and stability. PEG linkers typically range from 2 to 8 units in length. However, they have minimal light absorption and are relatively complex to synthesize.

### SUMMARY

The present disclosure reveals for the first time the use of VAGGFG as an enzymatically cleavable linker.

A ligand-drug conjugate of a general formula TL-(L-D)n, or a pharmaceutically acceptable salt or solvate thereof is provided,
where, TL is a targeting ligand, L is a linker unit, D is Eribulin, and n is a positive integer from 1 to 20; and
L contains a short peptide VAGGFG as an enzymatically cleavable linker.

The present disclosure also relates to a method for preparing the ligand-drug conjugate, a pharmaceutical composition, and a medical use thereof.

The newly discovered linker according to the present disclosure significantly improves drug release efficiency, has a stronger ability of killing tumor cells, and therefore, has promising potential in applications involving ligand-drug conjugates.

### BRIEF DESCRIPTION OF DRAWINGS

To describe the technical solutions in the specific embodiments of the present disclosure or in the prior art more clearly, the following briefly describes the accompanying drawings required for describing the specific embodiments or the prior art. Apparently, the accompanying drawings in the following description show merely some embodiments of the present disclosure, and persons of ordinary skill in the art may still derive other drawings from these accompanying drawings without creative efforts.
FIG. 1 shows viability test results of cells treated with antibodies 7B7-H04 and 7B7 conjugated with Eribulin via different linker units provided in an embodiment of the present disclosure.
FIG. 2 shows drug-to-antibody ratios (DARs) and purity results of antibodies 7B7-H04 and 7B7 conjugated with Eribulin via different linker units provided in an embodiment of the present disclosure;
   A: DAR test result of naked antibody 7B7;
   B: DAR test result of 7B7-H04 MC-VA-PAB-Eribulin;
   C: SEC-HPLC purity test result of 7B7-H04 MC-VA-PAB-Eribulin;
   D: DAR test result of 7B7-H04 MC-GGFG-PAB-Eribulin;
   E: SEC-HPLC purity test result of 7B7-H04 MC-GGFG-PAB-Eribulin;
   F: DAR test result of 7B7-H04 MC-VAGGFG-PAB-Eribulin;
   G: SEC-HPLC purity test result of 7B7-H04 MC-VAGGFG-PAB-Eribulin;
   H: DAR test result of 7B7 MC-VAGGFG-PAB-Eribulin; and
   I: SEC-HPLC purity test result of 7B7 MC-VAGGFG-PAB-Eribulin.

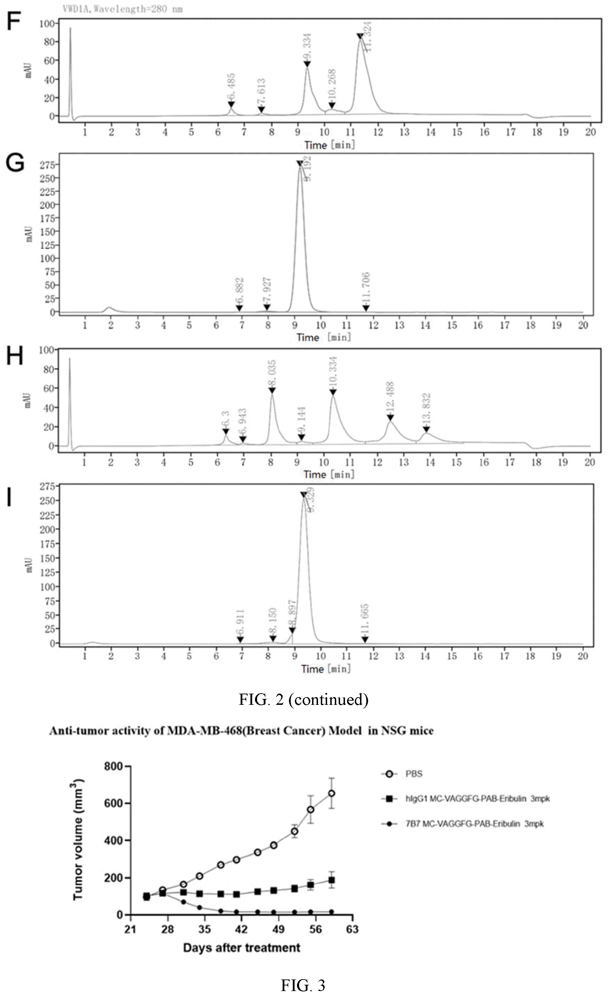

FIG. 3 shows the results of *in vivo* anti-tumor experiments using antibodies 7B7 conjugated with Eribulin via different linker units provided in an embodiment of the present disclosure.

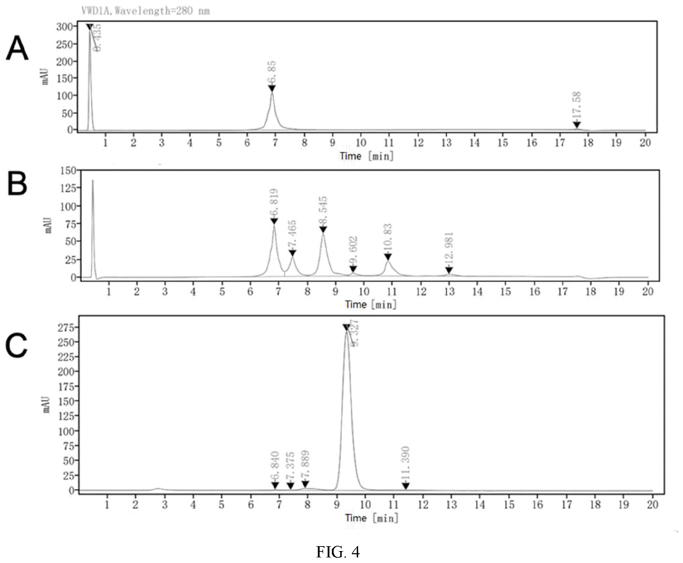

FIG. 4 shows the DAR and SEC-HPLC purity result of antibody Trastuzumab conjugated with MC-VAGGFG-PAB-Eribulin provided in an embodiment of the present disclosure;
   A: DAR test result of Trastuzumab naked antibody;
   B: DAR test result of Trastuzumab MC-VAGGFG-PAB-Eribulin; and
   C: SEC-HPLC purity test result of Trastuzumab MC-VAGGFG-PAB-Eribulin.

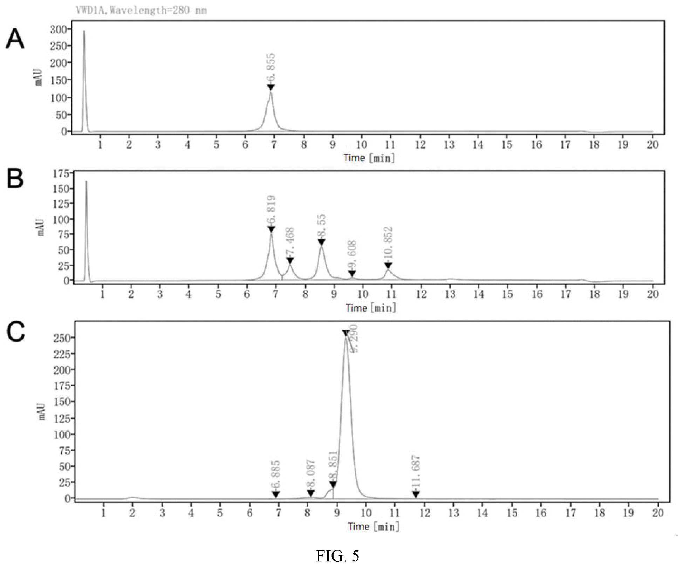

FIG. 5 shows the DAR and SEC-HPLC purity result of antibody Bemarituzumab conjugated with MC-VAGGFG-PAB-Eribulin provided in an embodiment of the present disclosure;
   A: DAR test result of Bemarituzumab naked antibody;
   B: DAR test result of Bemarituzumab MC-VAGGFG-PAB-Eribulin; and
   C: SEC-HPLC purity test result of Bemarituzumab MC-VAGGFG-PAB-Eribulin.

### DETAILED DESCRIPTION

Detailed references to embodiments of the present invention are now provided, with one or more examples described below. Each example is provided as an explanation rather than a limitation of the invention. In fact, it is apparent to those skilled in the art that various modifications and variations can be made to the invention without departing from its scope or spirit. For example, features illustrated or described as part of one embodiment can be used in another embodiment to create further embodiments.

Unless otherwise specified, all terms (including technical and scientific terms) used to disclose the invention have the same meanings as commonly understood by persons of ordinary skill in the art to which the invention belongs. For additional clarity, the following definitions are provided to better understand the teachings of the invention. The terms used herein in describing the invention are only to explain specific examples, and are not intended to limit the invention.

The selection range of the term "and/or" as used herein includes any one of two or more related listed items, as well as any and all combinations of the related listed items. These combinations include any two related listed items, any greater number of related listed items, or a combination of all the related listed items. It should be noted that when at least three items are conjoined by "and/or", it should be understood that in this application, the technical solution undoubtedly includes a solution where all items are connected using "logical and" as well as a solution where all items are connected using "logical or". For example, "A and/or B" includes three parallel solutions: A, B, and both A and B. For another example, technical solutions of "A, B, C and/or D" include any one of A, B, C or D (namely, a technical solution conjoined by "logical or"), and also include a combination of any one and all of A, B, C and D, that is, a combination of any two or three of A, B, C and D and a combination of four of A, B, C and D (namely, technical solutions conjoined by "logical and").

The terms "comprise", "include", and "contain" used herein are synonymous, and are inclusive or open-ended, and do not exclude additional uncited members, elements, or method steps.

Numerical ranges defined by endpoints in the present disclosure include all values, fractions, and the cited endpoints within the range.

Concentration values involved in the present disclosure may fluctuate within a specific range. For example, fluctuations can occur within the corresponding accuracy range. For instance, a concentration of 2% may allow fluctuations within a range of 0.1%. For larger values or values that do not require fine-tuned control, it is permitted for their meaning to include greater fluctuations. For example, 100 mM may allow fluctuations within ranges such as ±1%, ±2%, or ±5%. As for molecular weight, its meaning permits fluctuations of ±10%.

In the present disclosure, terms such as "multiple" and "various" refer to a quantity of two or more, unless otherwise specified.

In the present disclosure, technical features described in an open-ended manner include both closed-ended technical solutions consisting of the listed features and open-ended technical solutions that include the listed features.

In the present disclosure, the terms "preferred", "better", "preferable", and "desirable" are only intended to describe embodiments or examples with better effects, and should not be construed as a limitation on the protection scope of the present disclosure. In the present disclosure, "optionally" and "optional" indicate that an element may or may not be present, meaning it refers to one of the two parallel solutions, "present" or "absent". If a technical solution contains multiple instances of "optionally", unless otherwise stated and in the absence of contradictions or mutually restrictive relationships, each "optionally" is independent.

As used herein, the term "Eribulin" refers to a synthetic analog of Halichondrin B (a macrocyclic compound originally isolated from *Halichondria okadais),* with CAS number 253128-41-5. Eribulin is a microtubule dynamics inhibitor that is believed to bind to tubulin and inhibit mitotic spindle assembly, causing cell cycle arrest in the G2/M phase. The term "Eribulin mesylate", with CAS number 441045-17-6, refers to the mesylate salt of Eribulin, which is marketed under the brand name Halaven^{™}.

All the documents referenced in the present disclosure are cited herein as references, just as if each document were individually cited as a reference. Unless they conflict with the objectives and/or technical solutions of the present disclosure, the referenced documents in the present disclosure are incorporated in their entirety and for all purposes. When referencing documents in the present disclosure, the relevant technical features, terms, nouns, phrases, etc., as defined in the referenced documents, are also included by reference. When referencing documents in the present disclosure, examples and preferred embodiments of the relevant technical features in the referenced documents may also be incorporated into the present disclosure as references, but only to the extent that they can be implemented in the present disclosure. It should be understood that if there is any conflict between the referenced content and the description in the present disclosure, the present disclosure takes precedence, or the referenced content is adaptively amended based on the descriptions in the present disclosure.

The present disclosure involves the use of VAGGFG as an enzymatically cleavable linker.

Short peptides that can be used also include X¹X²GGFG, where X¹ and X² are both amino acid residues, and X¹ and X² are each independently selected from glycine, alanine, valine, leucine, isoleucine, proline, phenylalanine, tyrosine, tryptophan, serine, threonine, cysteine, methionine, aspartic acid, glutamine, asparagine, lysine, arginine, glutamic acid and histidine. In some embodiments, X¹X² is any one of VC, VA, AC, AV, AA, and VV. Further, any amino acid in X¹X²GGFG may be substituted by one or more substituents selected from halogen, hydroxyl, cyano, amino, alkyl, chlorinated alkyl, deuterated alkyl, alkoxy, and cycloalkyl.

The present disclosure also relates to a ligand-drug conjugate of a general formula TL-(L-D)n, or a pharmaceutically acceptable salt or solvate thereof,
where, TL is a targeting ligand, L is a linker unit, D is Eribulin, and n is a positive integer from 1 to 20; and
L includes a short peptide VAGGFG as an enzymatically cleavable linker.

Additionally, the value of n can be 2, 3, 4, 5, 6, 7, 8, 9, 10, 11, 12, 13, 14, 15, 16, 17, 18, or 19, preferably, 1 to 8, more preferably, 4 to 8.

The term "targeting ligand" refers to a macromolecular compound capable of specifically recognizing and binding to antigens or receptors associated with target cells. The role of the ligand is to deliver drugs to the target cell population that binds to the ligand. These ligands include, but are not limited to, protein hormones, lectins, growth factors, antibodies, binding-capable polypeptides, or other molecules capable of binding to cells. In the embodiments of the present disclosure, the targeting ligand is represented as TL, and the targeting ligand can form a bond with the linker unit through the heteroatom on the ligand.

The term "pharmaceutically acceptable salt" refers to the salt form of the ligand-drug conjugate disclosed herein, which is acceptable for administration to patients (e.g., mammals). Specifically, it is a salt that includes a counterion with acceptable safety for mammals under a given dosing regimen. Such salts can be derived from pharmaceutically acceptable inorganic or organic bases, as well as from pharmaceutically acceptable inorganic or organic acids. The ligand-drug conjugate disclosed herein contains at least one amino group, allowing it to form salts with acids. Non-limiting examples of pharmaceutically acceptable salts include: hydrochloride, hydrobromide, hydroiodide, sulfate, bisulfate, citrate, acetate, succinate, ascorbate, oxalate, nitrate, sorbate, hydrophosphate, dihydrogen phosphate, salicylate, hydrogen citrate, tartrate, maleate, fumarate, formate, benzoate, methanesulfonate, ethanesulfonate, benzenesulfonate, and p-toluenesulfonate. In particular, when the conjugated drug is Eribulin, the preferred pharmaceutically acceptable salt is Eribulin mesylate.

The term "solvate" used herein refers to a combination of the compound in the present disclosure and solvent molecules formed by solvation. In some cases, the solvate refers to a hydrate, that is, the solvent molecules are water molecules, and the compound in the present disclosure is combined with water to form a hydrate.

In some embodiments, the linker unit is -L¹-L²-L³-L⁴-, where L¹ is connected to TL, and L⁴ is connected to D;
where,
L¹ is selected from: 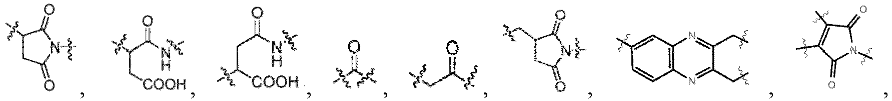 or a group required for click chemistry (preferably, 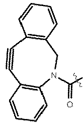 );
L² is selected from:
-NC(R¹R²)C(O), -NR³(CH₂)ₒC(O)-, -NR³(CH₂CH₂O)ₒCH₂C(O)-, -S(CH₂)ₚC(O)-, and a chemical bond, where o is an integer selected from 0 to 20; and p is an integer selected from 0 to 20;
R¹ and R² are each independently selected from hydrogen, deuterium, alkyl, substituted alkyl, deuterated alkyl, heteroalkyl, carboxyl, amino, and substituted amino;
R³ is selected from hydrogen, deuterium, halogen, alkyl, substituted alkyl, deuterated alkyl, cycloalkylalkyl, alkoxyalkyl, aryl, substituted aryl, and heteroaryl;
L¹ and L² share a nitrogen atom;
L³ is VAGGFG;
L⁴ is selected from -NR⁴(CR⁵R⁶)_{q}-, -C(O)NR⁴-, -C(O)NR⁴(CH₂)_{q}-, and a chemical bond, where q is an integer selected from 0 to 6; and
R⁴, R⁵, and R⁶ are each independently selected from hydrogen, deuterium, halogen, alkyl, substituted alkyl, deuterated alkyl, cycloalkyl, cycloalkylalkyl, alkoxyalkyl, a heterocyclic group, aryl, substituted aryl, and heteroaryl.

In some embodiments, L⁴ may also be selected from -NR⁴-aryl-(CR⁵R⁶)_{q}-OC(O)- and -NR⁴(CR⁵R⁶)_{q}-OC(O)-, where q is an integer selected from 0 to 6; and
R⁴, R⁵, and R⁶ are each independently selected from hydrogen, deuterium, halogen, alkyl, substituted alkyl, deuterated alkyl, cycloalkyl, cycloalkylalkyl, alkoxyalkyl, a heterocyclic group, aryl, substituted aryl, and heteroaryl.

In some embodiments, o may be 0, 1, 2, 3, 4, 5, 6, 7, 8, 9, 10, 11, 12, 13, 14, 15, 16, 17, 18, 19, or 20.

In some embodiments, p may be 0, 1, 2, 3, 4, 5, 6, 7, 8, 9, 10, 11, 12, 13, 14, 15, 16, 17, 18, 19, or 20.

In some embodiments, q may be 0, 1, 2, 3, 4, 5, or 6.

"L¹ and L² share a nitrogen atom" means that when the defined terminal group on the right side of the L¹ group and the defined terminal group on the left side of the L² group both contain a nitrogen atom, the two share the same nitrogen atom.

Obviously, L¹ can be simply replaced with other groups to conjugate with TL at other natural amino acid sites, unnatural amino acid sites, terminal sites, glycosylation sites, or through chelation or click chemistry. Examples of click chemistry conjugation include cycloaddition reactions, nucleophilic ring-opening reactions, non-alcohol-aldehyde carbonyl chemistry, or addition reactions of carbon-carbon multiple bonds.

In some instances, TL contains the functional group F¹, and L¹ has or is its complementary functional group. Preferably, the reactive group L¹ and the functional group F¹ can react in a bioorthogonal reaction, as these reactions do not interfere with biomolecules present during the process. Bioorthogonal reactions and suitable functional groups are known to those skilled in the art, such as those described in Gong and Pan, Tetrahedron Lett. 2015, 56, 2123-2132, including Staudinger ligation and copper-free click chemistry. Therefore, L¹ is preferably selected from 1,3-dipole, alkyne, (hetero)cyclooctyne, cyclooctene, tetrazine, ketone, aldehyde, alkoxyamine, hydrazine, and triphenylphosphine. For example, when F¹ is an azide group, the connection between azide-modified antibodies and linker-conjugates is preferably carried out via cycloaddition reactions. In this case, the functional group L¹ is preferably an alkynyl group, preferably a terminal alkynyl group or (hetero)cycloalkynyl group. For example, when F¹ is a ketone group, the connection between ketone-modified antibodies and linker-conjugates is preferably carried out through selective conjugation with hydroxylamine derivatives or hydrazine, respectively producing oximes or hydrazones. In this case, the functional group L¹ is preferably a primary amino group (for example, an -NH₂ group), an aminooxy group (for example, -O-NH₂), or a hydrazine group (for example, -N(H)NH₂). For example, when F¹ is an alkynyl group, the connection between alkyne-modified antibodies and linker-conjugates is preferably carried out via cycloaddition reactions, preferably 1,3-dipolar cycloaddition reactions. In this case, the functional group L¹ is preferably a 1,3-dipole, for example, azide, nitrone, or nitrile oxide. The positions of F¹ and L¹ may also be interchangeable.

The term "alkyl" refers to a saturated aliphatic hydrocarbon group, which is a linear or branched group including 1 to 20 carbon atoms, preferably, an alkyl group including 1 to 12 carbon atoms, more preferably, an alkyl group including 1 to 10 carbon atoms, and most preferably, an alkyl group including 1 to 6 carbon atoms. Non-limiting examples include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched isomers thereof, and the like. More preferably, the alkyl group is a lower alkyl group including 1 to 6 carbon atoms, and non-limiting examples include methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. The alkyl group may be substituted or non-substituted, and when the alkyl group is substituted, the substituent group can be substituted at any available attachment point. The substituents are preferably one or more of the following groups, independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkyloxy, cycloalkylthio, heterocycloalkylthio, and oxo.

The term "substituted alkyl" refers to an alkyl group in which a hydrogen atom has been replaced by a substituent group. Unless otherwise specified in the text, the substituents on the alkyl group can be selected from the following groups: -halogen, -OR', -NR'R", -SR', -SiR'R"R‴, -OC(O)R', -C(O)R', -CO₂R', -CONR'R", -OC(O)NR'R", -NR"C(O)R', -NR'-C(O)NR"R"', -NR"C(O)₂R', -NH-C(NH₂)=NH, -NR'C(NH₂)=NH, -NH-C(NH₂)=NR', -S(O)R', -S(O)₂R', -S(O)₂NR'R", -NR'S(O)₂R", -CN and -NO₂. The number of substituents can range from 0 to (2m'+1), where m' is the total number of carbon atoms in the group. R', R", and R‴ each independently refer to hydrogen, unsubstituted C₁-C₈ alkyl, unsubstituted aryl, aryl substituted by 1-3 halogen atoms, unsubstituted C₁-C₈ alkyl, C₁-C₈ alkoxy, C₁-C₈ thioalkoxy, or unsubstituted aryl-C₁₋₄ alkyl. When R' and R" are attached to the same nitrogen atom, they can, together with the nitrogen atom, form a 3-, 4-, 5-, 6-, or 7-membered ring. For instance, -NR'R" includes 1-pyrrolidinyl and 4-morpholinyl.

The term "heteroalkyl" refers to an alkyl group including one or more heteroatoms selected from N, O, and S, where the alkyl group is as defined above.

The term "alkoxy" refers to -O- (alkyl) and -O- (non-substituted cycloalkyl), where the definitions of alkyl or cycloalkyl are as described above. Non-limiting examples of alkoxy groups include: methoxy, ethoxy, propoxy, butoxy, cyclopropoxy, cyclobutoxy, cyclopentoxy, and cyclohexyloxy. Alkoxy groups can be either optionally substituted or unsubstituted. When substituted, the substituents are preferably one or more of the following groups, independently selected from alkyl, alkenyl, alkynyl, alkoxy, alkylthio, alkylamino, halogen, sulfhydryl, hydroxyl, nitro, cyano, cycloalkyl, heterocycloalkyl, aryl, heteroaryl, cycloalkoxy, heterocycloalkoxy, cycloalkylthio, and heterocycloalkylthio.

The term "alkoxyalkyl" refers to an alkyl group substituted with one or more alkoxy groups, preferably substituted with one alkoxy group, where the alkyl group is as defined above and the alkoxy group is as defined above.

The term "cycloalkyl" refers to a saturated or partially unsaturated monocyclic or polycyclic hydrocarbon substituent. Cycloalkyl rings contain 3 to 20 carbon atoms, preferably 3 to 12 carbon atoms, more preferably 3 to 10 carbon atoms, and most preferably 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl groups include spirocyclic, fused, and bridged cycloalkyl groups.

The term "heterocyclic group" refers to a saturated or partially unsaturated monocyclic or polycyclic hydrocarbon substituent including 3 to 20 ring atoms, where one or more of the ring atoms are heteroatoms selected from nitrogen, oxygen, and S(O)ₘ (m is an integer from 0 to 2). However, the ring structures do not include segments with -O-O-, -O-S-, or -S-S-. The remaining ring atoms are carbon. Preferably, the heterocyclic group contains 3 to 12 ring atoms, with 1, 2, 3, or 4 being heteroatoms; more preferably, it contains 3 to 10 ring atoms. Non-limiting examples of monocyclic heterocyclic groups include pyrrolidinyl, piperidinyl, piperazinyl, morpholinyl, thiomorpholinyl, homopiperazinyl, and the like. Polycyclic heterocyclic groups include spirocyclic, fused, and bridged heterocyclic groups.

The term "cycloalkylalkyl" refers to an alkyl group substituted with one or more cycloalkyl groups, preferably substituted with one cycloalkyl group, where the alkyl group is as defined above and the cycloalkyl group is as defined above.

The term "aryl" refers to an all-carbon monocyclic or fused polycyclic (i.e., rings sharing adjacent pairs of carbon atoms) group containing 6 to 14 carbon ring atoms with a conjugated π-electron system. Aryl groups can be monocyclic or polycyclic (i.e., they can contain more than one ring). In the case of polycyclic aromatic rings, only one ring in the polycyclic system is required to be aromatic, while the other rings can be saturated, partially saturated, or unsaturated. Preferred aryl groups are 6- to 10-membered, such as phenyl and naphthyl. The aryl ring can be fused to a heteroaryl ring, a heterocyclic ring, or a cyclic ring, where the ring connected to the parent structure is the aromatic ring.

The term "heteroaryl" refers to a heteroaromatic system containing 1 to 4 heteroatoms and 5 to 14 ring atoms, where the heteroatoms include oxygen, sulfur, or nitrogen. Heteroaryl groups are preferably 5- to 10-membered, and more preferably 5- or 6-membered. Examples include furanyl, thienyl, pyridyl, pyrrolyl, N-alkyl pyrrolyl, pyrimidinyl, pyrazinyl, imidazolyl, tetrazolyl, oxazolyl, and isoxazolyl. The heteroaryl ring can be fused to an aryl ring, a heterocyclic ring, or a cyclic ring, where the ring connected to the parent structure is the heteroaryl ring. Heteroaryl groups can be optionally substituted or unsubstituted.

The term "deuterated alkyl" refers to an alkyl group in which one or more hydrogen atoms are replaced by deuterium atoms, where alkyl is as defined above.

The term "halogen" refers to fluorine, chlorine, bromine, or iodine.

"Substituted" refers to one or more hydrogen atoms in the group, preferably up to 5, more preferably 1, 2, or 3 hydrogen atoms, being independently replaced by substituents. Substituents only occupy their possible chemical positions, which can be determined by those skilled in the art without undue effort (either experimentally or theoretically). For example, amino or hydroxyl groups with free hydrogen atoms may be unstable when combined with carbon atoms having unsaturated bonds (e.g., double bonds).

In some embodiments, -L¹-L²- is 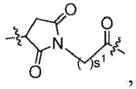 and s1 is 2, 3,4, 5, 6, 7, or 8.

In some embodiments, L⁴ is a p-aminobenzyloxycarbonyl (PAB) group.

In some embodiments, the targeting ligand is an antibody or an antigen-binding fragment thereof.

As used herein, the term "antibody" is used in its broadest sense and includes immunoglobulin or another type of molecule that contains one or more antigen-binding domains that specifically bind to an antigen, namely, a protein or peptide that exhibits binding specificity for a particular antigen. Specific examples of antibodies may include intact antibodies (for example, classic four-chain antibody molecules), single-chain antibodies, single-domain antibodies, bispecific antibodies, multispecific antibodies, and the like. A classic antibody molecule is usually a tetramer composed of two identical heavy chains and two identical light chains connected to each other through disulfide bonds. Based on a conservative difference in amino acid sequences, heavy and light chains are divided into variable regions (V) located at amino terminals and constant regions (C) located at carboxyl terminals. The variable region is used to recognize and bind the antigen, and the constant region (for example, Fc fragment) is used to initiate a downstream effect, for example, cell-mediated antibody-dependent cytotoxicity (ADCC) effect. Within the variable regions of the heavy chain and light chain, three local regions have a higher degree of variation in amino acid composition and sequences, are key binding positions for antibodies and antigens, and therefore, are also referred to as complementarity-determining regions (CDRs). Amino acid sequences of the CDRs can be readily determined by using numbering schemes recognized in the field, for example, Kabat, Chothia, IMGT, AbM or Contact. The antibodies can be IgG, IgM, IgD, IgE, or IgA antibodies.

An "antigen-binding fragment" of an antibody refers to an amino acid fragment in the antibody molecule that participates in specific antigen binding, for example, one of F(ab')₂, Fab and scFv.

The term "F(ab')₂" is obtained by digesting the entire full-length antibody via pepsins and removing most of the Fc region while maintaining some hinge regions intact. The F(ab')₂ fragment has two antigen-binding Fab portions bound together by disulfide bonds, and therefore, the F(ab')₂ fragment is a bivalent antibody. Taking F(ab')₂ prepared with IgG antibody, the molecular weight is about 110 kDa.

The term "Fab" is an antibody structure that can still bind to an antigen, and is monovalent and does not contain the Fc portion. After digestion of the full-length antibody by papain, two Fab fragments and one Fc fragment are obtained, and each Fab fragment is approximately 50 kDa.

The term "scFv" is formed by binding the heavy chain variable region and the light chain variable region of the antibody into one peptide chain via short peptides. Through proper folding, the variable regions from the heavy and light chains interact with each other via non-covalent bonds to form the Fv segment, and therefore, scFv can better maintain affinity activity against antigens.

In some embodiments, the antibody or the antigen-binding fragment thereof is selected from a rabbit-derived antibody, a murine antibody, a chimeric antibody, a humanized antibody, or a fully human antibody.

The "rabbit-derived antibody/murine antibody" refers to an antibody whose variable and constant regions (if present) are derived from rabbit/mouse immunoglobulin sequences. The rabbit-derived antibody/murine antibody can be conveniently obtained by immunizing rabbits/rats (including mice or rats) with the corresponding antigen and isolating the target antibody therefrom. Alternatively, the rabbit-derived antibody/murine antibody can be obtained by isolating and culturing cells (for example, B cells) expressing the target antibody after immunization of the rabbits/rats with the corresponding antigen. Alternatively, after immunization of the rabbits/rats with the corresponding antigen, cells expressing the target antibody are isolated, cultured, and fused with immortalized cells such as myeloma cells to obtain hybridoma cells, and culturing the hybridoma cells can obtain a large amount of target antibodies (for example, monoclonal antibodies) over a long term.

The term "chimeric antibody" is an antibody obtained by fusing a variable region of the first animal-derived antibody with a constant region of the second animal-derived antibody. To create the chimeric antibody, a hybridoma that secretes the first animal-derived specific monoclonal antibody needs to be first created, then the variable region gene in the hybridoma cell is cloned, then the constant region gene of the second animal-derived antibody is cloned as required, the first animal-derived variable region gene is ligated with the second animal-derived constant region gene into a chimeric gene, the chimeric gene is inserted into an expression vector, and finally, a chimeric antibody molecule is expressed in the eukaryotic system or the prokaryotic system. In a preferred embodiment in the present disclosure, the first animal origin is rabbits or rats, and the second animal origin is preferably humans, which can alleviate the immune response induced by the first animal-derived antibody. The light chain of the chimeric antibody further includes a light chain constant region of the human-derived κ and λ chains, or variants thereof. The heavy chain of the chimeric antibody further includes a heavy chain constant region of the human-derived IgG1, IgG2, IgG3, and IgG4 or variants thereof. Subtypes of the constant region of the antibody, isomers of different people, and mutations occurring based on changes in effects and functions of the constant region do not affect preparation of antibody conjugates. Linker toxin conjugation sites, for example cysteine, lysine, glutamine, carboxy-terminus of the peptide chain, and glycosylation sites, including native and engineered sites, are usually used for conjugation.

The term "humanized antibody", also known as CDR-grafted antibody, refers to an antibody produced by transplanting the first animal-derived CDR sequence into human antibody variable region frameworks, that is, different types of human germline antibody framework sequences. The humanized antibody can overcome a heterologous reaction induced because chimeric antibodies carry a large amount of first animal-derived protein components. Such framework sequences can be obtained from public DNA databases or published references containing germline antibody gene sequences. For example, germline DNA sequences of human heavy and light chain variable region genes are available in "VBase" human germline sequence database (www.mrccpe.com.ac.uk/vbase) and found in Sequences of Proteins of Immunological Interest (Kabat, E.A. et al., 1991, 5th ed.) To avoid both a decrease in immunogenicity and a resulting decrease in activity, a minimum of reverse mutation or back mutation can be performed on the human antibody variable region framework sequence to maintain activity. The humanized antibody in the present disclosure also includes a mature humanized antibody obtained after phage shows affinity to the CDR. In a preferred embodiment of the present disclosure, the first animal-derived source is rabbit-derived or murine-derived. The human antibody variable region framework is designed and selected. To prevent a decline in immunogenicity while avoiding a reduction in activity, minimal back mutations can be introduced into the human antibody variable region to maintain activity.

Fully humanized antibodies (human antibodies) refer to antibodies where human antibody genes are introduced into genetically engineered animals lacking antibody genes, using transgenic or chromosomal transfer technologies. These animals are then able to express human antibodies, achieving the goal of full humanization of the antibodies.

In some embodiments, the antibody is selected from anti-CD3 antibody, anti-FOLR1 antibody, anti-ROR1 antibody, anti-TNFα antibody, anti-tissue factor (TF) antibody, anti-EpCAM antibody, anti-EGFRvIII antibody, anti-DLL-3 antibody, anti-PSMA antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Claudin 6 antibody, anti-Claudin 9 antibody, anti-Claudin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 (ErbB3) antibody, anti-MUC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD70 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD20 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody, and anti-CD123 antibody.

In some specific embodiments, the antibody is selected from a group consisting of the following antibodies:
anti-GD2 antibody 3F8, Abagovomab, Abciximab, ACZ885 (canakinumab), Adalimumab, Adecatumumab, Afelimomab, Afutuzumab, Alacizumab pegol, Alemtuzumab, Altumomab pentetate, Anatumomab mafenatox, Anrukinzumab (IMA-638), Apolizumab, Arcitumomab, Aselizumab, Atezolizumab, Atorolimumab, Avelumab, Bapineuzumab, Basiliximab, Bavituximab, Bectumomab, Belimumab, Bertilimumab, Besilesomab, Bevacizumab, Biciromab, Bivatuzumab mertansine, Blinatumomab, Brentuximab vedotin, Briakinumab, Canakinumab, Cantuzumab mertansine, Capromab pendetide, Catumaxomab, Cedelizumab, Certolizumabpegol, Cetuximab, Citatuzumab bogatox, Cixutumumab, Clenoliximab, Clivatuzumab tetraxetan, CNTO148 (golimumab), CNTO 1275 (ustekinumab), Conatumumab, Dacetuzumab, Daclizumab, Denosumab, Detumomab, Dorlimomab aritox, Dorlixizumab, durvalumab, Ecromeximab, Eculizumab, Edobacomab, Edrecolomab, Efalizumab, Efungumab, Elsilimomab, Enlimomab pegol, Epitumomabcituxetan, Epratuzumab, Erlizumab, Ertumaxomab, Etaracizumab, Exbivirumab, Fanolesomab, Faralimomab, Felvizumab, Fezakinumab, Figitumumab, Fontolizumab, Foravirumab, Fresolimumab, Galiximab, Gavilimomab, Gemtuzumab ozogamicin, Golimumab, Gomiliximab, Ibalizumab, Ibritumomab tiuxetan, Igovomab, Imciromab, Infliximab, Intetumumab, Inolimomab, Inotuzumab ozogamicin, Ipilimumab, Iratumumab, Keliximab, Labetuzumab, Lebrikizumab, Lemalesomab, Lerdelimumab, Lexatumumab, Libivirumab, Lintuzumab, Lucatumumab, Lumiliximab, Mapatumumab, Maslimomab, Matuzumab, Mepolizumab, Metelimumab, Milatuzumab, Minretumomab, Mitumomab, Morolimumab, Motavizumab, Muromonab_CD3, MY0-029 (stamulumab), Nacolomab tafenatox, Naptumomab estafenatox, Natalizumab, Nebacumab, Necitumumab, Nerelimomab, Nimotuzumab, Nivolumab, Nofetumomab merpentan, Ocrelizumab, Odulimomab, Ofatumumab, Omalizumab, Oportuzumab monatox, Oregovomab, Otelixizumab, Pagibaximab, Palivizumab, Panitumumab, Panobacumab, Pascolizumab, Pembrolizumab, Pemtumomab, Pertuzumab, Pexelizumab, Pintumomab, Priliximab, Pritumumab, PRO 140, Rafivirumab, Ramucirumab, Ranibizumab, Raxibacumab, Regavirumab, Reslizumab, Rilotumumab, Rituximab, Robatumumab, Rontalizumab, Rovelizumab, Ruplizumab, Satumomab, Sevirumab, Sibrotuzumab, Sifalimumab, Siltuximab, Siplizumab, Solanezumab, Sonepcizumab, Sontuzumab, Stamulumab, Sulesomab, Tacatuzumab tetraxetan, Tadocizumab, Talizumab, Tanezumab, Taplitumomab paptox, Tefibazumab, Telimomab aritox, Tenatumomab, Teneliximab, Teplizumab, TGN1412, Ticilimumab, tremeIimumab, Tigatuzumab, TNX-355 (ibalizumab), TNX-650, TNX-901 (talizumab), Tocilizumab, Toralizumab, Tositumomab, Trastuzumab, Tremelimumab, Tucotuzumab celmoleukin, Tuvirumab, Urtoxazumab, Ustekinumab, Vapaliximab, Vedolizumab, Veltuzumab, Vepalimomab, Visilizumab, Volociximab, Votumumab, Zalutumumab, Zanolimumab, Ziralimumab, and Zolimomab aritox.

In some embodiments, the antibody is selected from:
(i) Trastuzumab antibody;
(ii) Bemarituzumab antibody; and
(iii) anti-B7-H3 antibody, where the heavy-chain complementarity-determining regions HCDR1, HCDR2, and HCDR3 of the anti-B7-H3 antibody are represented by SEQ ID NOs: 1 to 3, respectively, and the light-chain complementarity-determining regions LCDR1, LCDR2, and LCDR3 of the anti-B7-H3 antibody are represented by SEQ ID NOs: 4 to 6, respectively.

In some embodiments, the heavy chain variable region HCVR of the anti-B7-H3 antibody is represented by SEQ ID NO: 7, and the light chain variable region LCVR of the anti-B7-H3 antibody is represented by SEQ ID NO: 8.

In some embodiments, the heavy chain constant region of the anti-B7-H3 antibody is represented by SEQ ID NO: 9 or 10, and the light chain constant region of the anti-B7-H3 antibody is represented by SEQ ID NO: 11.

Variants of the above-mentioned amino acid sequences also fall within the scope of the present invention. The corresponding variants, compared to any of the polypeptides represented by SEQ ID NO:1 to SEQ ID NO:6, respectively contain up to 3 amino acid mutations in at least one CDR region. Alternatively, in comparison to the overall sequences of SEQ ID NO:7 to SEQ ID NO:15, the variants may contain up to 3 or more mutations. For instance, the variants may have a sequence with at least 80%, 85%, 90%, 93%, 95%, 97%, or 99% identity to any of the polypeptides represented by SEQ ID NO:7 to SEQ ID NO:15. The mutations may include substitutions, deletions, insertions, or any combination thereof. Preferably, the mutations are conservative substitutions.

"Conservative substitution" refers to substitution of an amino acid in the protein with another amino acid having similar characteristics (for example, charge, side chain size, hydrophobicity/hydrophilicity, backbone conformation, and rigidity), allowing frequent changes without changing biological activity of the protein.

The substitution generally regarded as conservative substitution is mutual substitution of aliphatic amino acids Ala, Val, Leu and Ile, exchange of hydroxyl residues Ser and Thr, exchange of acid residues Asp and Glu, exchange of amide residues Asn and Gln, exchange of basic residues Lys and Arg, and substitution between aromatic residues Phe and Tyr. Persons skilled in the art know that substitution of a single amino acid in a non-essential region of a peptide usually substantially does not change biological activity (Watson et al. (1987), Molecular Biology of the Gene, The Benjamin/Cummings Pub. Co., p. 224, (4th ed.)). In addition, substitution with a structurally or functionally similar amino acid is unlikely to disrupt biological activity.

In some embodiments, a structure of the ligand-drug conjugate is shown below:

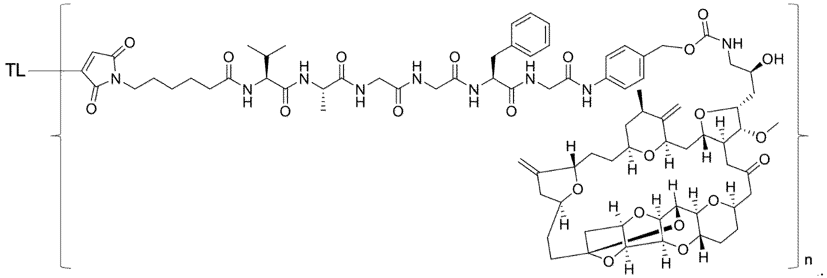

The present disclosure further relates to a method for preparing the foregoing ligand-drug conjugate or the pharmaceutically acceptable salt or solvate thereof, including:
after reduction of the targeting ligand, subjecting the targeting ligand to a conjugation reaction with pre-synthesized -L-D to obtain a compound represented by a general formula of TL-L-D.

The reducing agent is preferably TCEP, particularly for reducing the disulfide bond of the targeting ligand.

The present disclosure further relates to a pharmaceutical composition including the foregoing ligand-drug conjugate or the pharmaceutically acceptable salt or solvate thereof, and a pharmaceutically acceptable excipient, diluent, or carrier.

As used herein, the "pharmaceutically acceptable carrier, diluent, or excipient" includes any material that allows a component to remain biologically active when combined with the active component and that does not react with the immune system of the subject.

The present disclosure further relates to the use of the foregoing ligand-drug conjugate or the pharmaceutically acceptable salt or solvate thereof in preparation of a medicament for treating a tumor.

The term "cancer" refers to a physiological condition or disease characterized by dysregulated cell growth. "Tumor" includes cancer cells. In some embodiments, the tumor is a solid tumor or hematological tumor such as breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, lung cancer (for example, non-small cell lung cancer), colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, or leukemia.

The present disclosure also relates to a method for treating a medical condition of a subject, including: administering a safe and effective dose of the foregoing ligand-drug conjugate.
Preferably, the medical condition is cancer.

As used herein, the phrase "safe and effective dose" means that a dose of a compound or a composition is great enough to significantly effectively alleviate a symptom or a condition in treatment, but is small enough to avoid severe side effects (at a reasonable benefit-risk ratio) within a reasonable pharmaceutical regulation range. A safe and effective dose of an active component in the pharmaceutical composition used in method in the present disclosure varies along with factors including a specific treated symptom, an age and a physical condition of a treated patient, severity of a disease, duration of a treatment, a contemporaneous treatment, a specific used active component, a specific used pharmaceutically acceptable excipient, and knowledge and skills of the physician involved in the treatment.

It should be understood that the hypothetical treatment method also includes administering other therapeutic entities, particularly preferably immunotherapeutic entities, including viral cancer vaccines (for example, adenovirus vectors encoding cancer-specific antigens), bacterial cancer vaccines (for example, non-pyrogenic E. coli expressing one or more cancer-specific antigens), yeast cancer vaccines, N-803 (or referred to as ALT-803, Altor BioScience), chemotherapeutic drugs, antibodies (bound to, for example, tumor-associated antigens or patient-specific tumor neoantigens), stem cell grafts (for example, allogeneic or autologous), and tumor-targeted cytokines (for example, NHS-IL12, where IL-12 is conjugated with tumor-targeted antibodies or fragments thereof). In some embodiments, the hypothetical treatment method also includes: subjecting the patient to the radiotherapy. In some embodiments, the hypothetical treatment method also includes: subjecting the patient to a surgery such as a surgery to remove a tumor.

The ligand-drug conjugate can also be administered and/or formulated in conjunction with antivirals, antibiotics, analgesics, corticosteroids, steroids, oxygen, antioxidants, COX inhibitors, cardioprotectant agents, metal chelators, IFN-γ, and/or NSAIDs. The pharmaceutical composition can include the foregoing therapeutic entities.

The terms "subject" and "patient" are interchangeable herein and refer to any animals such as any mammals, including (but not limited to) humans, non-human primates, rodents, dogs, cats, chimpanzees, orangutans, gibbons, macaques, marmosets, pigs, horses, pandas, and elephants.

As used herein, "treatment" refers to any improvement in consequences of any disease, for example prolonged survival time, reduced morbidity, and/or reduced side effects of by-products as alternative treatments. As is easily understood in the art, complete eradication of the disease is preferred, but unnecessary for therapeutic behavior. As used herein, "treatment" may involve curing, healing, alleviating, mitigating, altering, remedying, improving, reducing, ameliorating, reversing, or influencing a disease, symptoms of a disease, or a predisposition to diseases such as cancer.

As it is known to persons skilled in the art, the pharmaceutical composition in the present disclosure can be administered via any route. In some embodiments, the pharmaceutical composition in the present disclosure is administered intravenously (IV).

The embodiments of the present disclosure are described in detail below with reference to examples: It should be understood that these examples are only used to illustrate the present disclosure and are not intended to limit the scope of the present disclosure. In the following examples, for a test method for which a specific condition is not specified, preferably, refer to the guidance provided in the present disclosure, a test manual or a conventional condition in the art, another test method known in the art, or a condition recommended by a manufacturer.

In the following specific examples, a measurement parameter related to an ingredient may have a slight deviation within a weighing accuracy range unless otherwise specified. For temperature and time parameters, an acceptable deviation due to instrument test accuracy or operation accuracy is allowed.

### Example 1 Preparation of Anti-B7-H3 Antibody

Based on the anti-B7-H3 antibody sequence disclosed in Chinese patent application CN113402610A, which is published on September 17, 2021, further expression and purification of the monoclonal antibody were conducted.

Unless otherwise specified, the expression of all antibodies disclosed herein can be performed using the commercial vector pTT5, with the antibody sequence inserted between the EcoR I and Hind III restriction enzyme sites located downstream of its promoter. The following sequence could be selected as the nucleic acid sequence, including the signal peptide, before the light or heavy chain sequence of the antibody:

GAATTCGCCGCCACCATGGGATGGTCCTGTATTATCCTGTTCCTGGTCGCTACCGCTACC
GGTGTCCACTCA

The underlined portions of the sequence correspond to the EcoR I and the starting amino acids.

The antibody light chain or heavy chain sequence is followed by:
TGAAAGCTT (stop codon and Hind III).

The antibody amino acid sequence is optimized to its nucleotide sequence before being inserted between the above signal peptide and terminator.
Heavy chain H00 sequence: 
EVQLVESGGGLVKPGGSLRLSCAASGFTFSSYAVSWVRQAPGKGLEWVASISGGGIYIYYPD
SVKGRFTISRDNAKNSLYLQMNSLRAEDTAVYYCARHGGAGYFDYWGQGTLVTVSS (SEQ
ID NO: 7)
 



 


 (SEQ ID NO: 9)
Light chain L00 sequence: 
DSQMTQSPSSLSASVGDRVTITCRGSESVHSYLAWYQQKPGKAPKLLVYNAKTLQSGVPSR
FSGSGSGTDFTLTISSLQPEDFATYYCQQHYGSPPWTFGGGTKVEIK (SEQ ID NO: 8)
 

 (SEQ ID NO: 11)
Heavy chain H04 sequence:

EVQLVESGGGLVKPGGSLRLSCAASGFTFSSYAVSWVRQAPGKGLEWVASISGGGIYIYYPD
SVKGRFTISRDNAKNSLYLQMNSLRAEDTAVYYCARHGGAGYFDYWGQGTLVTVSS (SEQ
ID NO: 7)

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTSPPSPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVCTLPPCRDELTKNQV
SLTCLVKGFYPSDIAVEWESNGQPENNYKTTPPVLDSDGSFFLYSKLTVDKSRWQQGNVFSC
SVMHEALHNHYTQKSLSLSPGK (SEQ ID NO: 10)

The anti-B7-H3 antibody corresponds to clone 7B7. The antibody with the light chain L00 and heavy chain H00 is referred to as 7B7, while the antibody with the light chain L00 and heavy chain H04 is referred to as 7B7-H04. The 7B7-H04 antibody is an engineered antibody, optimized for producing conjugates with uniform drug-to-antibody ratios, making it highly suitable for evaluating different linker-toxin conjugates.

The light and heavy chains of the above-mentioned antibodies were separately constructed into vectors, and the plasmids were extracted. Suspended, acclimated CHO-K1 cells were resuscitated in OPM-CD TransCHO medium (manufacturer: OPM, product code: P83059) and cultured to a density of 2 million cells/ml with a viability above 95%, in a volume of 1000 ml. The light chain plasmid (0.5 mg) and heavy chain plasmid (0.5 mg) were combined and dissolved in 10 ml of culture medium. 3 mg of PEI (manufacturer: Polysciences, product code: 24765-1) was dissolved in 10 ml of culture medium. The plasmid and PEI solutions were mixed and left at room temperature for 10 minutes, then added dropwise to the 1000 ml cell culture. The culture was incubated at 37°C for 5 days, after which the supernatant was collected by centrifugation at 12,000 g for 15 minutes. The supernatant was purified using a HiTrap Mabselect SuRe column and eluted with 50 mM acetic acid. The collected peak was neutralized and transferred to PBS (pH 7.4) using a 30 kDa ultrafiltration tube (manufacturer: Merck, product code: UFC9030). The antibody concentration was determined by measuring the absorbance value at 280 nm, which was divided by the theoretical extinction coefficient to calculate the concentration.

### Example 2 Synthesis of Linker-Toxin in Control Group

### 1. Synthesis of Linker-Toxin MC-VA-PAB-Eribulin

The designed linker-toxin MC-VA-PAB-Eribulin (LK-322018) has the following structure:

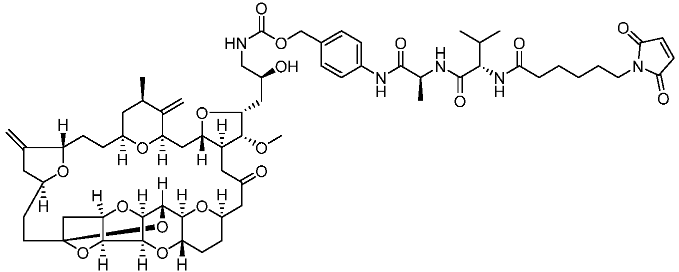

The synthesis route of compound LK-322018 is as follows:

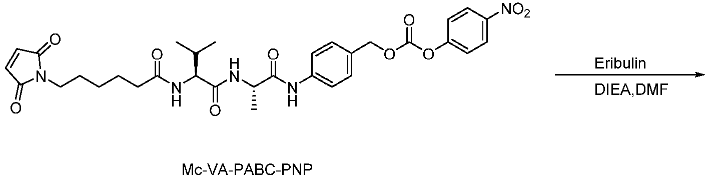

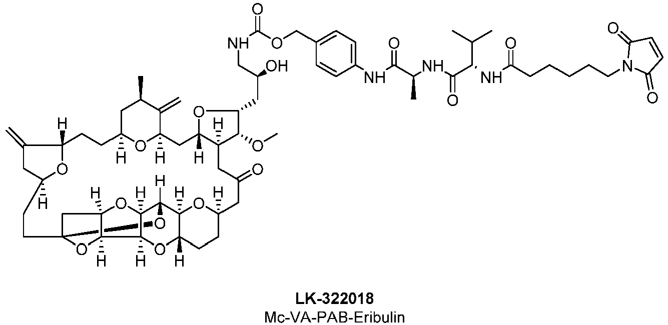

Synthesis of compound LK-322018: The commercially available compound MC-VA-PABC-PNP (5.9 mg, 0.091 mmol) was dissolved in 1 ml of N,N-dimethylformamide. N,N-diisopropylethylamine (3.1 mg, 0.024 mmol) and the commercially available Eribulin (5 mg, 0.007 mmol) were sequentially added. The reaction was carried out at room temperature for 16 hours, and the completion of the reaction was monitored via liquid chromatography-mass spectrometry (LC-MS). After the reaction was complete, 5 ml of water was added, and ethyl acetate extraction was performed three times (10 ml each). The organic phases were separated, combined, washed with saturated saline, dried with anhydrous sodium sulfate, and concentrated. The crude product was purified using preparative liquid chromatography to yield LK-322018. ESI-MS m/z: 1240 (M-H).

### 2. Synthesis of Linker-Toxin MC-GGFG-PAB-Eribulin

The designed linker-toxin MC-GGFG-PAB-Eribulin (LK-322016) has the following structure:

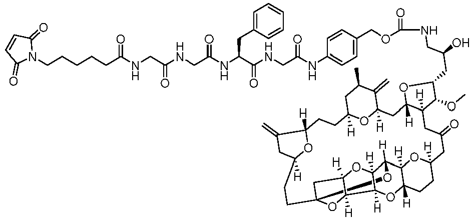

The synthesis route of compound LK-322016 is as follows:

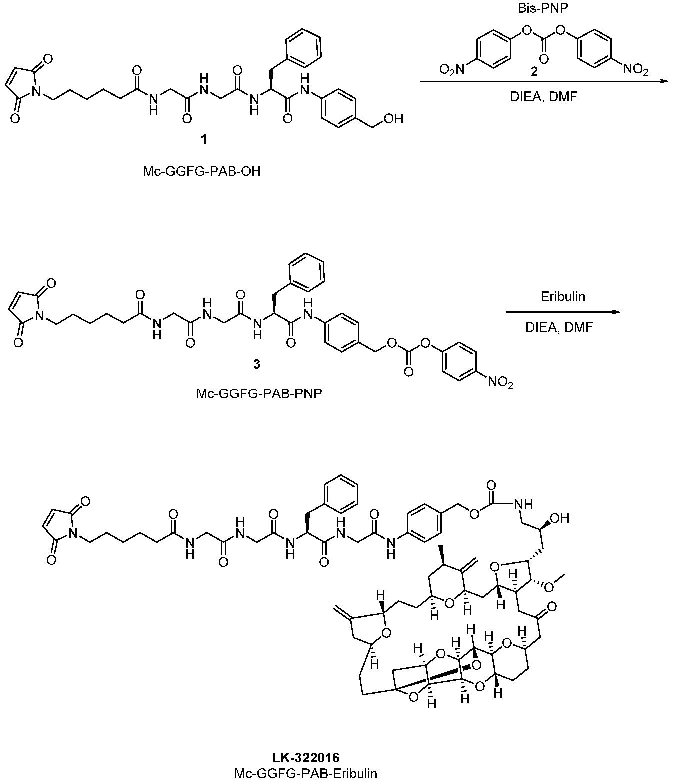

Synthesis of compound 3: The commercially available compound 1 (50 mg) was dissolved in 2 ml of N,N-dimethylformamide. Bis-PNP (36 mg, 0.1182 mmol) and N,N-diisopropylethylamine (20.3 mg, 0.006053 mmol) were sequentially added. The reaction was carried out at room temperature for 16 hours, and the completion of the reaction was monitored via LC-MS. After the reaction was complete, 15 ml of water was added, and ethyl acetate extraction was performed three times (10 ml each). The organic phases were separated, combined, washed with saturated saline, dried with anhydrous sodium sulfate, and concentrated. The crude product was purified using preparative liquid chromatography to yield compound 3. ESI-MS m/z: 743 (M+H).

Synthesis of compound LK-322016: Compound 3 (7.26 mg, 0.009079 mmol) was dissolved in 1 mL of N,N-dimethylformamide. N,N-diisopropylethylamine (3.1 mg, 0.02412 mmol) and the commercially available Eribulin (5 mg, 0.007 mmol) were sequentially added. The reaction was carried out at room temperature for 16 hours, and the completion of the reaction was monitored via LC-MS. After the reaction was complete, 5 ml of water was added, and ethyl acetate extraction was performed three times (10 ml each). The organic phases were separated, combined, washed with saturated saline, dried with anhydrous sodium sulfate, and concentrated. The crude product was purified using preparative liquid chromatography to yield LK-322016. ESI-MS m/z: 1389 (M-H).

### 3. Synthesis of Linker-Toxin Mal-PEG2-VC-PAB-Eribulin

The synthesis steps referred to page 145 of Chinese patent application CN108883198A, published on November 23, 2018. The synthesis method and procedure were identical to those in the patent. The study described in (DOI: 10.1158/1535-7163.MCT-17-1215, "MORAb-202, an Antibody-Drug Conjugate Utilizing Humanized Anti-human FRα Farletuzumab and the Microtubule-targeting Agent Eribulin, has Potent Antitumor Activity") demonstrated that Mal-PEG2-VC-PAB-Eribulin and MC-VC-PAB-Eribulin exhibited similar activity across various cell lines and were thus considered essentially equivalent. Consequently, Mal-PEG2-VC-PAB-Eribulin was used interchangeably with MC-VC-PAB-Eribulin in certain contexts within the present disclosure.

### Example 3 Synthesis of Linker-Toxin in Test Group

### Synthesis of Linker-Toxin MC-VAGGFG-PAB-Eribulin

The designed linker-toxin MC-VAGGFG-PAB-Eribulin (LK-322022) has the following structure:

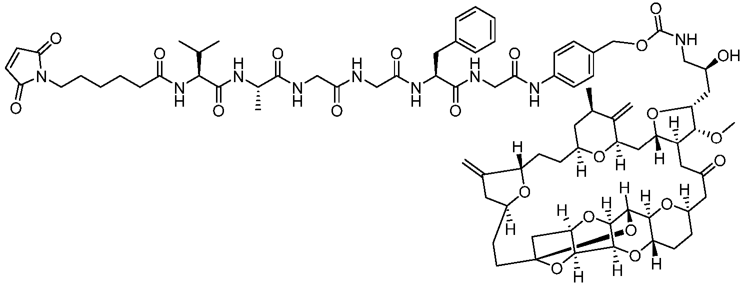

The synthesis route of compound LK-322022 is as follows:

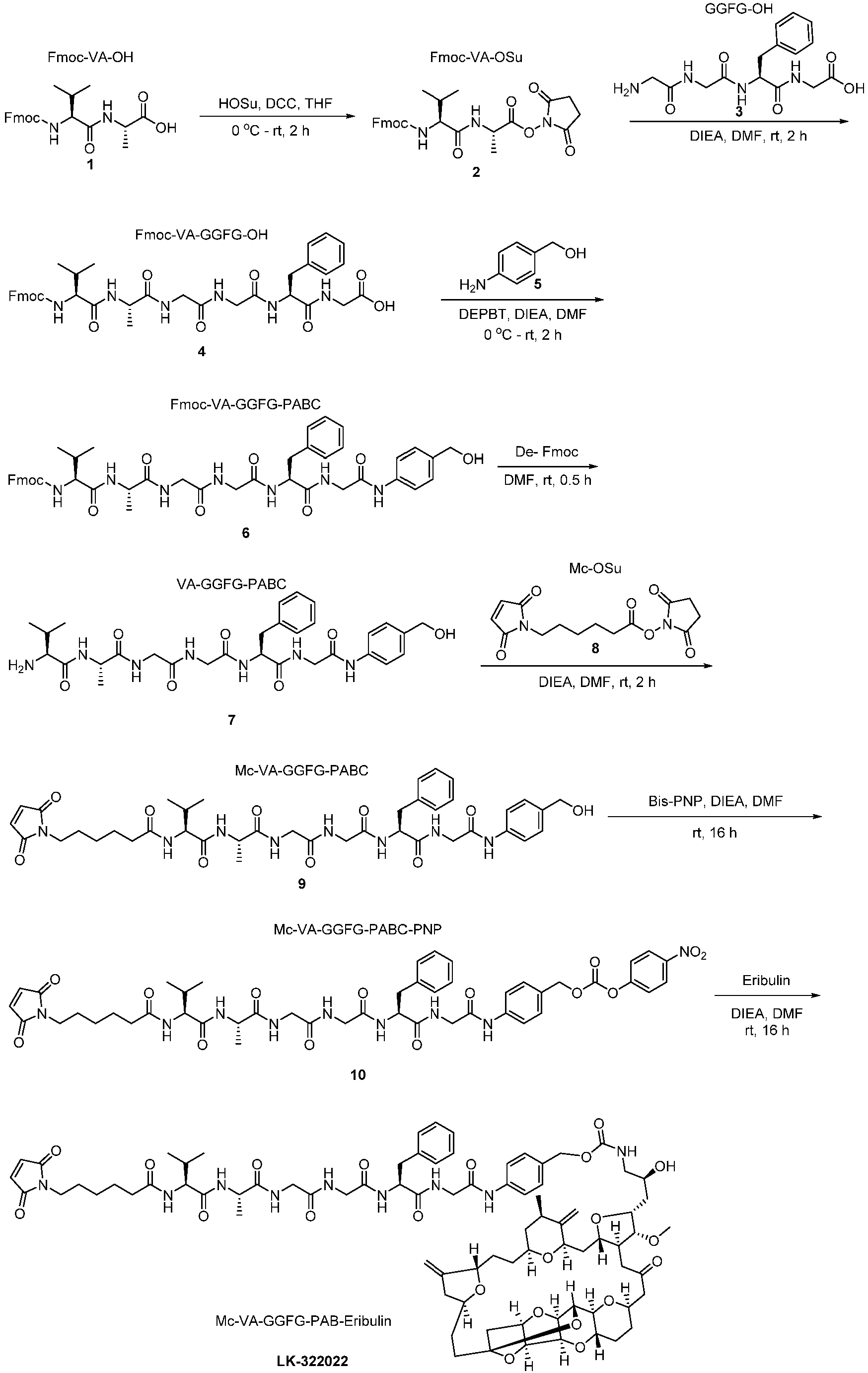

Synthesis of compound 2: The commercially available compound 1 (100 mg, 0.244 mmol) was dissolved in 3 mL of tetrahydrofuran, the resulting mixture was cooled to 0°C, HOSU (33.7 mg, 0.293 mmol) and DCC (60.3 mg, 0.293 mmol) were added, and the resulting mixture was allowed to react at room temperature for 2 hours. After monitoring the completion of the reaction using TLC and LC-MS, the reaction solution was filtered, and the filtrate was concentrated to obtain the crude product to be directly used for the next reaction. ESI-MS m/z: 508 (M+H).

Synthesis of compound 4: Compound 2 (crude, 0.244 mmol) was mixed with the commercially available compound 3 (82 mg, 0.244 mmol) in 3 mL of N,N-dimethylformamide. N,N-diisopropylethylamine (126 mg, 0.976 mmol) was added, and the resulting mixture was allowed to react at room temperature for 2 hours. After monitoring the completion of the reaction using LC-MS, the solvent was removed by distillation under reduced pressure, the crude product was pulped with 10 mL of water, and the resulting mixture was filtered, to obtain a crude product of the title compound (200 mg, yield: 100%). ESI-MS m/z: 729 (M+H).

Synthesis of compound 6: Compound 4 (200 mg, 0.274 mmol) was dissolved in 5 mL of N,N-dimethylformamide, the resulting mixture was cooled to 0°C, and compound 5 (41 mg, 0.329 mmol), N,N-diisopropylethylamine (107 mg, 0.823 mmol) and DEPBT (124 mg, 0.412 mmol) were added sequentially, and the resulting mixture was allowed to react at room temperature for 4 hours. After monitoring the completion of the reaction using LC-MS, the reaction solution was quenched with 10 mL of solution of ammonium chloride in water, ethyl acetate extraction was performed three times (10 mL each time), organic phases were combined after liquid separation, washed with saturated brine, dried with anhydrous sodium sulfate and then concentrated, and the obtained crude product was purified via silica gel column chromatography [dichloromethane/methanol = 88:12 (v/v)] to obtain the title compound 6 (100 mg, yield: 43.8%). ESI-MS m/z: 834 (M+H).

Synthesis of compound 7: Compound 6 (100 mg, 0.120 mmol) was dissolved in 3 mL of N,N-dimethylformamide, piperidine (31 mg, 0.360 mmol) was added, and the resulting mixture was allowed to react at room temperature for 0.5 hours. After monitoring the completion of the reaction using LC-MS, the solvent was removed by distillation under reduced pressure, and the obtained crude product was purified via silica gel column chromatography [dichloromethane/methanol = 82:18 (v/v)] to obtain the title compound 7 (76 mg, yield: 100%). ESI-MS m/z: 612 (M+H).

Synthesis of compound 9: Compound 7 (76 mg, 0.124 mmol) was mixed with the commercially available compound 8 (46 mg, 0.149 mmol) in 3 mL of N,N-dimethylformamide, N,N-diisopropylethylamine (32 mg, 0.248 mmol) was added, and the resulting mixture was allowed to react at room temperature for 2 hours. After monitoring the completion of the reaction using LC-MS, the solvent was removed by distillation under reduced pressure, and the obtained crude product was purified via silica gel column chromatography [dichloromethane/methanol = 82:18 (v/v)] to obtain the title compound 9 (70 mg, yield: 70%). ESI-MS m/z: 805 (M+H).

Synthesis of compound 10: Compound 9 (70 mg, 0.087 mmol) was dissolved in 3 mL of N,N-dimethylformamide, Bis-PNP (40 mg, 0.130 mmol) and N,N-diisopropylethylamine (22 mg, 0.174 mmol) were added sequentially, and the resulting mixture was allowed to react at room temperature for 16 hours. After monitoring the completion of the reaction using LC-MS, the solvent was removed by distillation under reduced pressure, and the obtained crude product was purified via silica gel column chromatography [dichloromethane/methanol = 90:10 (v/v)] to obtain the compound 10 in the title (40 mg, yield: 47.6%). ESI-MS m/z: 970 (M+H).

Synthesis of compound LK-322022: Compound 10 (14 mg, 0.014 mmol) was dissolved in 1 mL of N,N-dimethylformamide, N,N-diisopropylethylamine (3.5 mg, 0.027 mmol) and commercially available Eribulin (5 mg, 0.007 mmol) were added sequentially, and the resulting mixture was allowed to react at room temperature for 16 hours. After monitoring the completion of the reaction using LC-MS, 5 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain LK-322022 (5.1 mg, yield: 48.1%). ESI-MS m/z: 1560 (M-H).

### Example 4 Antibody Conjugation

The antibodies 7B7-H04 and 7B7 from the above examples, each weighing 3 mg, were diluted to 3 mg/ml using 10 mM sodium phosphate buffer (pH 7.4). A stock solution of tris(2-carboxyethyl)phosphine hydrochloride (TCEP) was added. The TCEP stock solution was prepared in 10 mM sodium phosphate buffer (pH 7.4) at a concentration of 10 mM (TCEP manufacturer: Sigma, product code: C4706-2G, CAS: 51805-45-9). The final molar ratio of TCEP to antibody was set at 4:1. The antibody was reduced at 37°C for 45 minutes. The linker-toxin stock solutions from Examples 2 and 3 were added. These stock solutions were prepared by dissolving the linker-toxin in DMSO at a concentration of 5 mM. The final molar ratio of linker-toxin to antibody was set at 6:1. The antibody was incubated at 2 to 8°C for conjugation for 2 hours. The antibody was then transferred to a 30 kDa ultrafiltration tube (manufacturer: Merck, product code: UFC9030) and subjected to centrifugation at 3000 g at 2 to 8°C to exchange the solution. Residual toxin was reduced to one-thousandth of the reaction concentration. The antibody was subsequently sterilized by filtration, its concentration was measured, and it was aliquoted and frozen for storage.

To obtain products with different DARs, the molar ratio of antibody : reductant : linker-toxin was adjusted within the range of 1 : 1-6 : 2-15, while all other conditions remained unchanged. The DAR value of the final conjugated product was determined based on the test results.

### Example 5 In Vitro Comparison of Different ADCs' Effects

### I. Test Method

### 1. Test of Cell Viability

Human Calu-6 cells (manufacturer: Procell, product code: CL-327, human lung cancer cells) were resuscitated and seeded into a 96-well culture plate. The culture medium used was DMEM/F12 (manufacturer: Hyclone, product code: SH30023.01), supplemented with 10% FBS (manufacturer: Gibco, product code: 10099141). Each well contained 2,500 cells in 150 µl of medium. The plate was incubated at 37°C in a 5% CO₂ incubator. Approximately 20 hours later, 50 µl of medium containing different antibody-drug conjugates were added to each well, setting the final drug concentrations at 0 to 100 nM across 12 dilution gradients (initial concentration: 100 nM; subsequent 5-fold serial dilutions across 10 gradients; last gradient containing no drug, and each sample was tested in duplicate wells). The plate was co-incubated for 4 to 7 days, and then 50 µl of CTG detection reagent (CellTiter-Glo, manufacturer: Promega, product code: G7575) was added to each well. After a 2-minute reaction, fluorescence intensity was measured using a Tecan Spark instrument, following the reagent kit's recommended protocol. The average value of duplicate wells was plotted on the Y-axis, while the log10 values of the dilution gradients were plotted on the X-axis to generate a smooth curve. EC/IC₅₀ values were calculated using four-parameter fitting.

A similar cell viability assay method was used to verify results by replacing Calu-6 cells with Jurkat, MCF-7, or MDA-MB-468 cell lines.

### 2. Test and Analysis of DAR

The average number of drugs conjugated with antibodies (DAR value) was analyzed using a hydrophobic interaction chromatography HIC-HPLC method. The analysis column used was TSKgel Butyl-NPR, 4.6 mm × 10 cm, 2.5 µm (manufacturer: TOSOH, product code: 042168). Approximately 0.3 mg of the test sample in 150 µL was mixed with 150 µL of mobile phase A and centrifuged at 10,000 g for 5 minutes. The supernatant was collected. Mobile phase A was 20 mM sodium phosphate + 1.5 M ammonium sulfate at pH 7.0, and mobile phase B was 20 mM sodium phosphate + 20% (v/v) acetonitrile at pH 7.0. The analysis column was connected to an Agilent 1260 high-performance liquid chromatograph with a flow rate of 0.6 mL/min. Mobile phase A was flushed for over 30 minutes to stabilize the UV baseline at 280 nm, followed by injection of 50 µg of sample. After flushing with mobile phase A for 2 minutes, an 18-minute gradient elution (0% B - 100% B) was performed, followed by a 5-minute wash with 100% B. The peak area percentages for each DAR value were calculated, and the average DAR value was obtained by summing the products of each percentage and DAR value, then dividing by the total percentage.

### 3. Test and Analysis of Purity

SEC-HPLC purity was determined using a size-exclusion chromatography method. The analysis column was TSKgel G3000SWXL, 7.8 mm × 30 cm, 5 µm (manufacturer: TOSOH, product code: 08541). Approximately 0.3 mg of the test sample in 300 µL was centrifuged at 10,000 g for 5 minutes, and the supernatant was collected. The mobile phase was 50 mM sodium phosphate + 0.1 M sodium chloride at pH 6.8. The analysis column was connected to an Agilent 1260 high-performance liquid chromatograph with a flow rate of 0.8 mL/min. The mobile phase was flushed for over 30 minutes to stabilize the UV baseline at 280 nm, followed by injection of 100 µg of sample. The mobile phase was flushed for 20 minutes. The peak area percentages of high molecular weight, monomer, and low molecular weight components were calculated.

### II. Test Results

### 1. Test Results of Cell Viability

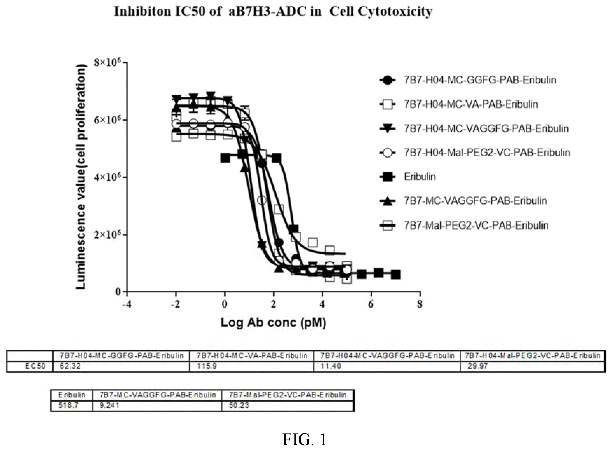

Antibody 7B7-H04 was conjugated with MC-VA-PAB-Eribulin, Mal-PEG2-VC-PAB-Eribulin, MC-GGFG-PAB-Eribulin, and MC-VAGGFG-PAB-Eribulin, and the activity of the conjugates against Calu-6 cell viability showed EC/IC₅₀ values of 115.9 pM, 29.97 pM, 62.32 pM, and 11.40 pM, respectively (Figure 1), indicating that MC-VAGGFG-PAB-Eribulin showed significantly better efficacy than the other groups.

For 7B7 conjugated with Mal-PEG2-VC-PAB-Eribulin and MC-VAGGFG-PAB-Eribulin, the EC/IC₅₀ values were 50.23 pM and 9.241 pM, respectively (Figure 1), with MC-VAGGFG-PAB-Eribulin still demonstrating superior technical performance.

The EC/IC₅₀ trends observed in other cell lines besides Calu-6 were similar, with MC-VAGGFG-PAB-Eribulin consistently exhibiting the best efficacy.

### 2. Results of DAR and Purity

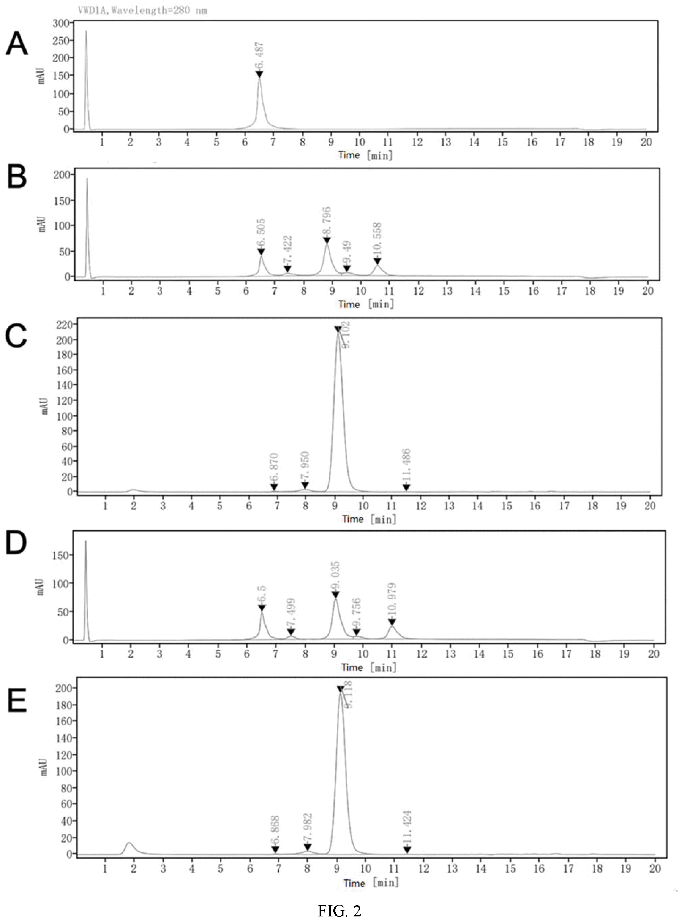

The average DAR values and SEC-HPLC purity results for each group are shown in Figure 2 and the table below:

| **Group** | **Average DAR value** | **SEC-HPLC purity (%)** |
|---|---|---|
| **7B7 naked antibody** | 0 | - |
| **7B7-H04 MC-VA-PAB-Eribulin** | 3.33 | 97.15 |
| **7B7-H04 MC-GGFG-PAB-Eribulin** | 3.27 | 97.10 |
| **7B7-H04 MC-VAGGFG-PAB-Eribulin** | 3.31 | 98.66 |
| **7B7 MC-VAGGFG-PAB-Eribulin** | 4.25 | 95.88 |
| **7B7-H04 Mal-PEG2-VC-PAB-Eribulin** | <4.2 | >95 |

The average DAR values of the conjugates were 3.33, 3.27, and 3.31 (FIG. 2), indicating that the VAGGFG peptide linker exhibited better activity compared to GGFG and VA peptide linkers when the DARs were similar.
Using methods similar to those mentioned above, validations were conducted in several common tumor cell lines (e.g., MCF-7 and MDA-MB-468), revealing consistent trends. Conjugates containing the VAGGFG linker demonstrated optimal EC/IC₅₀ performance compared to other conjugates. Furthermore, the average DAR value and SEC-HPLC purity results remained stable, meeting the requirements for antibody-drug conjugates.

### Example 6 In Vivo Pharmacodynamic Analysis

The *in vivo* pharmacodynamic evaluation was conducted by Bioduro (www.bioduro-sundia.com). Well-cultured human breast cancer tumor cell lines (MDA-MB-468) were inoculated into immunodeficient mice (B-NDG) at a rate of 10 million cells per mouse. When tumor growth reached 100 mm³, the mice were randomly divided into groups. The next day, each group was administered a cumulative dose of 3 mg/kg twice, once per week, and tumor size was measured twice a week until the end of the experiment. The entire process was approved by the Experimental Animal Committee.

As shown in Figure 3, the results indicated that the 7B7 MC-VAGGFG-PAB-Eribulin group effectively inhibited tumor volume.

Using the same animal model, the cell lines were replaced with Calu-6 and MCF-7 for further validation. Each group independently received doses ranging from 1 to 10 mg/kg (e.g., 2, 3, 4, 5, 6, 7, 8, or 9 mg/kg), and the trend was consistent with the MDA-MB-468 experiment, demonstrating optimal tumor volume inhibition..

### Example 7 Stability Test

The stability of the 7B7 MC-VAGGFG-PAB-Eribulin conjugate was tested in PBS buffer, cynomolgus monkey plasma, and mouse plasma at a concentration of 100 nM. The samples were incubated at 37°C for 0, 4, 7, 10, and 14 days, and the cytotoxic activity of the samples against Jurkat cells was measured. It was observed that the release rate of Eribulin (Y-axis) increased over time (X-axis), with a maximum release of 0.1 to 5%. This indicated excellent plasma stability of the linker.

### Example 8 Conjugation Test of Other Antibodies

The experimental results of the foregoing examples were verified based on antibodies 7B7-H04 and 7B7.
The heavy chain sequence of Trastuzumab: 
EVQLVESGGGLVQPGGSLRLSCAASGFNIKDTYIHWVRQAPGKGLEWVARIYPTNGYTRYA
DSVKGRFTISADTSKNTAYLQMNSLRAEDTAVYYCSRWGGDGFYAMDYWGQGTLVTVSS
 
ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVDKSRWQQGNVF
SCSVMHEALHNHYTQKSLSLSPGK (SEQ ID NO: 12)
The light chain sequence of Trastuzumab: 
DIQMTQSPSSLSASVGDRVTITCRASQDVNTAVAWYQQKPGKAPKLLIYSASFLYSGVPSRF
SGSRSGTDFTLTISSLQPEDFATYYCQQHYTTPPTFGQGTKVEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 13)
The heavy chain sequence of Bemarituzumab: 
QVQLVQSGAEVKKPGSSVKVSCKASGYIFTTYNVHWVRQAPGQGLEWIGSIYPDNGDTSY
NQNFKGRATITADKSTSTAYMELSSLRSEDTAVYYCARGDFAYWGQGTLVTVSSASTKGPS
VFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNVFSCSVMHE
ALHNHYTQKSLSLSPGK (SEQ ID NO: 14)
The light chain sequence of Bemarituzumab:

DIQMTQSPSSLSASVGDRVTITCKASQGVSNDVAWYQQKPGKAPKLLIYSASYRYTGVPSR
FSGSGSGTDFTFTISSLQPEDIATYYCQQHSTTPYTFGQGTKLEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 15)

Further, following the above examples, antibody-drug conjugates (ADCs) of Trastuzumab and Bemarituzumab conjugated with the linker-toxin MC-VAGGFG-PAB-Eribulin were prepared for validation. The results showed an average DAR of 2 to 3 and a purity >95% (Figures 4 and 5). Activity was measured using the SK-BR-3 cell line (human breast cancer cells) and KATO III cell line (human gastric cancer cells). Using the same method as for Calu-6 cells, the EC/IC₅₀ values for cell viability were determined to be 32 pM and 97 pM, respectively.

### Example 9 Conjugation Test of Other Drugs

To test the differences among various toxins, different peptide-linker-toxins were synthesized, and conjugates were prepared and tested for activity following the experimental methods of the above examples. The results showed that, at the cellular level, there were no significant differences among the tested peptide-linkers when using Monomethyl Auristatin E (MMAE) or Exatecan as toxins, differing from the results using Eribulin as a toxin in the above examples.

The compound MC-VC-PAB-MMAE is commercially available, and previous reports indicate no significant differences between MC-VA-PAB-MMAE and MC-VC-PAB-MMAE.

The synthesis route of the compound MC-GGFG-PAB-MMAE is as follows:

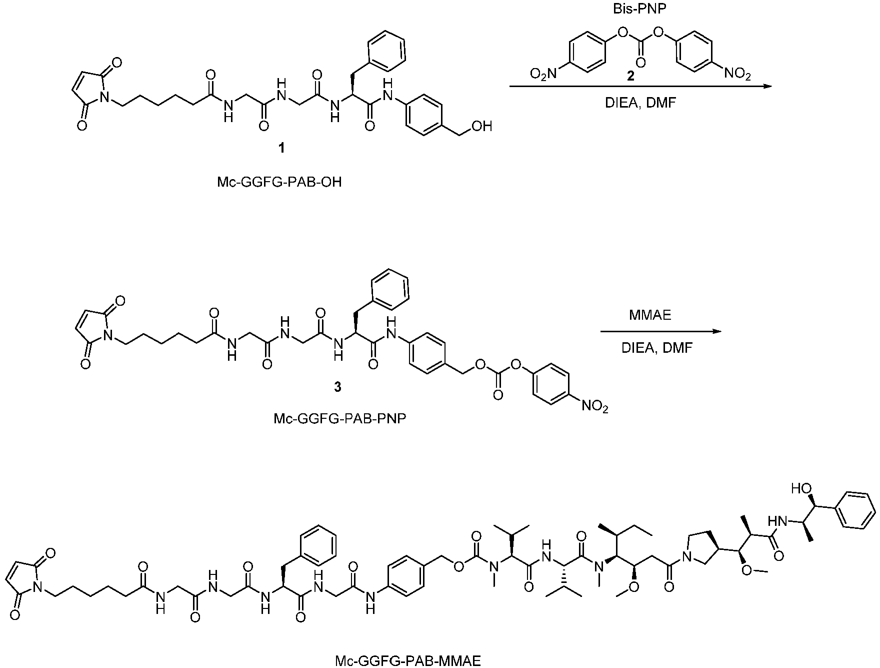

Synthesis steps: The commercially available compound 1 (50 mg, 0.0865 mmol) was dissolved in 2 mL of N,N-dimethylformamide, Bis-PNP (36 mg, 0.1182 mmol) and N,N-diisopropylethylamine (20.3 mg, 0.006053 mmol) were added sequentially, and the resulting mixture was allowed to react at room temperature for 16 hours. After monitoring the completion of the reaction using LC-MS, 15 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain the compound 3 (50 mg, yield: 77.8%). The compound 3 (7.26 mg, 0.009 mmol) was dissolved in 1 mL of N,N-dimethylformamide, N,N-diisopropylethylamine (3.1 mg, 0.02412 mmol) and commercially available MMAE (7.9 mg, 0.011 mmol) were added sequentially, and the resulting mixture was allowed to react at room temperature for 15 hours. After monitoring the completion of the reaction using LC-MS, 5 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain the target compound (8.5 mg, yield: 62%). ESI-MS m/z: 1378 (M+H).

The synthesis route of the compound MC-VAGGFG-PAB-MMAE is as follows:

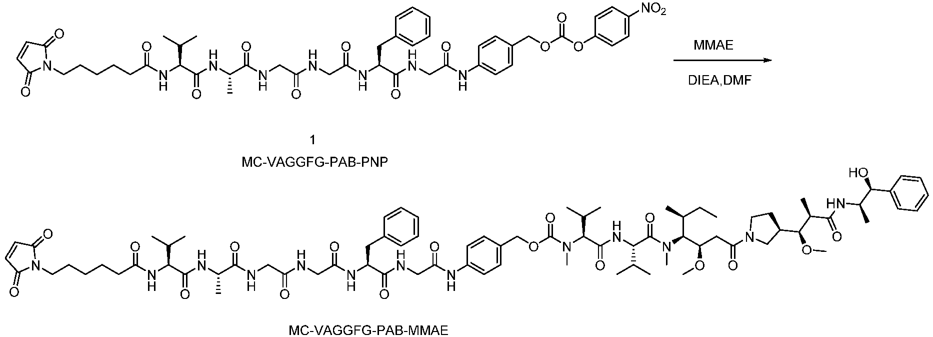

Steps: Compound 1 (the synthesis route was the same as that of the test group) (9.7 mg, 0.01 mmol) was dissolved in 1 mL of N,N-dimethylformamide, N,N-diisopropylethylamine (3.0 mg, 0.02 mmol) and commercially available MMAE (7.9 mg, 0.011 mmol) were added sequentially, and the resulting mixture was allowed to react at room temperature for 15 hours. After monitoring the completion of the reaction using LC-MS, 5 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain the target compound (9.3 mg, yield: 60%). ESI-MS m/z: 1548 (M+H).

The compound MC-VA-PAB-Exatecan is commercially available.

The synthesis route of the compound MC-GGFG-PAB-Exatecan is as follows:

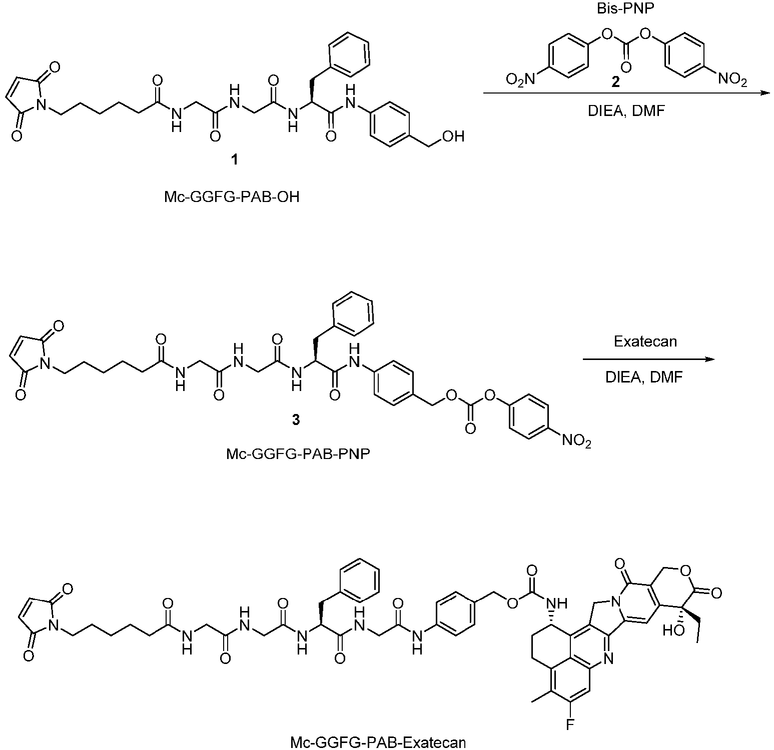

Steps: The commercially available compound 1 (50 mg, 0.0865 mmol) was dissolved in 2 mL of N,N-dimethylformamide, Bis-PNP (36 mg, 0.1182 mmol) and N,N-diisopropylethylamine (20.3 mg, 0.006053 mmol) were added sequentially, and the resulting mixture was allowed to react at room temperature for 16 hours. After monitoring the completion of the reaction using LC-MS, 15 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain compound 3 (50 mg, yield: 77.8%). Compound 3 (7.26 mg, 0.009079 mmol) was dissolved in 1 mL of N,N-dimethylformamide, N,N-diisopropylethylamine (3.1 mg, 0.02412 mmol) and commercially available Exatecan (5 mg, 0.011 mmol) were added sequentially, and the resulting mixture was allowed to react at room temperature for 15 hours. After monitoring the completion of the reaction using LC-MS, 5 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain the target compound (6.0 mg, yield: 61%). ESI-MS m/z: 1096 (M+H).

The synthesis route of the compound MC-VAGGFG-PAB-Exatecan is as follows:

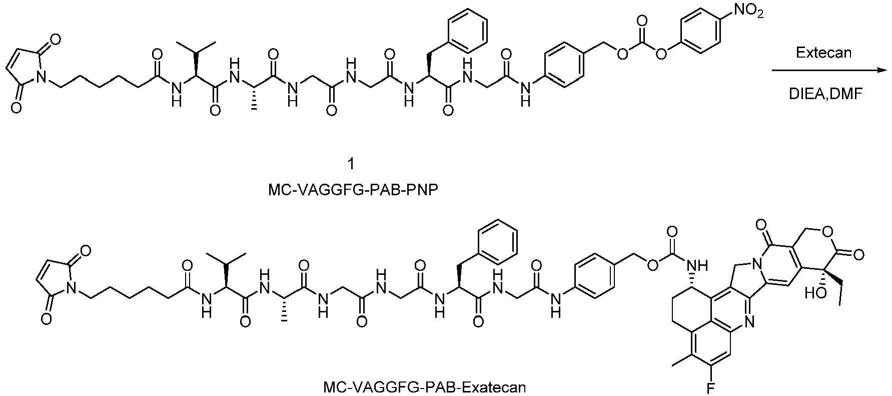

Steps: Compound 1 (the synthesis route was the same as that of the test group) (9.7 mg, 0.01 mmol) was dissolved in 1 mL of N,N-dimethylformamide, N,N-diisopropylethylamine (3.0 mg, 0.02 mmol) and commercially available Exatecan (5 mg, 0.011 mmol) were added sequentially, and the resulting mixture was allowed to react at room temperature for 15 hours. After monitoring the completion of the reaction using LC-MS, 5 mL of water was added, ethyl acetate extraction was performed three times (10 mL each time), organic phases were combined after liquid separation, washed with saturated brine, dried with anhydrous sodium sulfate and then concentrated, and the obtained crude product was purified using preparative liquid chromatography to obtain the target compound (7.3 mg, yield: 58%). ESI-MS m/z: 1266 (M+H).

The compounds were conjugated with antibody 7B7 following the method described in Example 4 to prepare conjugates with a DAR of 3 to 4, and their activity against the MCF-7 cell line was tested according to the method in Example 5. The results showed that for conjugates with MMAE toxin, the IC₅₀ values were 0.49 nM, 0.41 nM, and 0.27 nM for VC, GGFG, and VAGGFG peptide linkers, respectively, while for conjugates with Exatecan toxin, the IC₅₀ values were 242 nM, 270 nM, and 307 nM for VA, GGFG, and VAGGFG peptide linkers, respectively.

The examples described above merely illustrate several embodiments of the present disclosure. Although the descriptions are relatively specific and detailed, they should not be understood as limiting the scope of the invention. It should be noted that, for those skilled in the art, various modifications and improvements can be made without departing from the essence of the present disclosure, all of which fall within the scope of protection provided by the present disclosure. Therefore, the scope of protection of the invention shall be defined by the appended claims, and the description and drawings may be used to interpret the content of the claims.

### EMBODIMENTS

1. Use of VAGGFG as an enzymatically cleavable linker.
2. A ligand-drug conjugate of a general formula TL-(L-D)n, or a pharmaceutically acceptable salt or solvate thereof,
   wherein TL is a targeting ligand, L is a linker unit, D is Eribulin, and n is a positive integer from 1 to 20; and
   L comprises a short peptide VAGGFG as an enzymatically cleavable linker.
3. The ligand-drug conjugate or the pharmaceutically acceptable salt or solvate thereof according to embodiment 2, wherein the linker unit is -L¹-L²-L³-L⁴-, in which L¹ is connected to TL, and L⁴ is connected to D,
   wherein,
   L¹ is selected from: 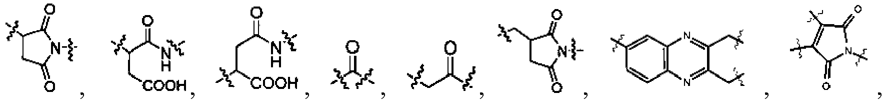 or a group required for click chemistry (preferably, 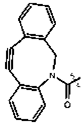 );
   L² is selected from:
      -NC(R¹R²)C(O), -NR³(CH₂)ₒC(O)-, -NR³(CH₂CH₂O)ₒCH₂C(O)-, -S(CH₂)ₚC(O)-, and a chemical bond, wherein o is an integer selected from 0 to 20; and p is an integer selected from 0 to 20;
      R¹ and R² are each independently selected from hydrogen atom, deuterium atom, alkyl, substituted alkyl, deuterated alkyl, heteroalkyl, carboxyl, amino, and substituted amino;
      R³ is selected from hydrogen atom, deuterium atom, halogen, alkyl, substituted alkyl, deuterated alkyl, cycloalkylalkyl, alkoxyalkyl, aryl, substituted aryl, and heteroaryl;
      L¹ and L² share a nitrogen atom;
      L³ is VAGGFG;
      L⁴ is selected from -NR⁴(CR⁵R⁶)_{q}-, -C(O)NR⁴-, -C(O)NR⁴(CH₂)_{q}-, and a chemical bond, wherein q is an integer selected from 0 to 6; and
      R⁴, R⁵, and R⁶ are each independently selected from hydrogen atom, deuterium atom, halogen, alkyl, substituted alkyl, deuterated alkyl, cycloalkyl, cycloalkylalkyl, alkoxyalkyl, a heterocyclic group, aryl, substituted aryl, and heteroaryl.
4. The ligand-drug conjugate or the pharmaceutically acceptable salt or solvate thereof according to embodiment 3, wherein -L¹-L²- is 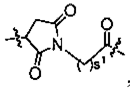 , and s1 is 2, 3, 4, 5, 6, 7 or 8.
5. The ligand-drug conjugate or the pharmaceutically acceptable salt or solvate thereof according to embodiment 2, wherein the linker unit is -L¹-L²-L³-L⁴-, wherein L¹ is connected to TL, and L⁴ is connected to D;
   wherein,
   L¹ is selected from: 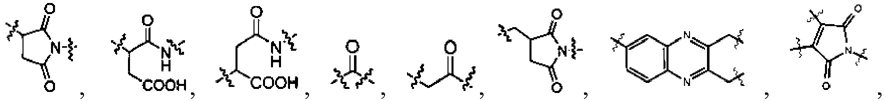 or a group required for click chemistry (preferably, 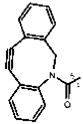 );
   L² is selected from:
      -NC(R¹R²)C(O), -NR³(CH₂)ₒC(O)-, -NR³(CH₂CH₂O)ₒCH₂C(O)-, -S(CH₂)ₚC(O)-, and a chemical bond, wherein o is an integer selected from 0 to 20; and p is an integer selected from 0 to 20;
      R¹ and R² are each independently selected from hydrogen atom, deuterium atom, alkyl, substituted alkyl, deuterated alkyl, heteroalkyl, carboxyl, amino, and substituted amino;
      R³ is selected from hydrogen atom, deuterium atom, halogen, alkyl, substituted alkyl, deuterated alkyl, cycloalkylalkyl, alkoxyalkyl, aryl, substituted aryl, and heteroaryl;
      L¹ and L² share a nitrogen atom;
      L³ is VAGGFG;
      L⁴ is selected from -NR⁴-aryl-(CR⁵R⁶)_{q}-OC(O)- and -NR⁴(CR⁵R⁶)_{q}-OC(O)-, wherein q is an integer selected from 0 to 6; and
      R⁴, R⁵, and R⁶ are each independently selected from hydrogen atom, deuterium atom, halogen, alkyl, substituted alkyl, deuterated alkyl, cycloalkyl, cycloalkylalkyl, alkoxyalkyl, a heterocyclic group, aryl, substituted aryl, and heteroaryl.
6. The ligand-drug conjugate or the pharmaceutically acceptable salt or solvate thereof according to embodiment 5, wherein -L¹-L²- is 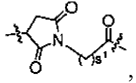 and s1 is 2, 3, 4, 5, 6, 7 or 8.
7. The ligand-drug conjugate or the pharmaceutically acceptable salt or solvate thereof according to embodiment 5, wherein L⁴ is a p-aminobenzyloxycarbonyl (PAB) group.
8. The ligand-drug conjugate or the pharmaceutically acceptable salt or solvate thereof according to any one of embodiments 2 to 7, wherein the targeting ligand is an antibody or an antigen-binding fragment thereof.
9. The ligand-drug conjugate or the pharmaceutically acceptable salt or solvate thereof according to embodiment 8, wherein the antibody or the antigen-binding fragment thereof is selected from a rabbit-derived antibody, a murine antibody, a chimeric antibody, a humanized antibody, and a fully human antibody.
10. The ligand-drug conjugate or the pharmaceutically acceptable salt or solvate thereof according to embodiment 9, wherein the antibody is selected from anti-CD3 antibody, anti-FOLR1 antibody, anti-ROR1 antibody, anti-TNFα antibody, anti-TF antibody, anti-EpCAM antibody, anti-EGFRvIII antibody, anti-DLL-3 antibody, anti-PSMA antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-Claudin 6 antibody, anti-Claudin 9 antibody, anti-Claudin 18.2 antibody, anti-Mesothelin antibody, anti-HER2 antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 antibody, anti-MUC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, anti-CD70 antibody, anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, anti-CD20 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody, and anti-CD123 antibody.
11. The ligand-drug conjugate or the pharmaceutically acceptable salt or solvate thereof according to embodiment 8, wherein the antibody is selected from:
   (i) Trastuzumab antibody;
   (ii) Bemarituzumab antibody; and
   (iii) anti-B7-H3 antibody, wherein heavy-chain complementarity-determining regions HCDR1, HCDR2, and HCDR3 of the anti-B7-H3 antibody are represented by SEQ ID NOs: 1 to 3, respectively, and light-chain complementarity-determining regions LCDR1, LCDR2, and LCDR3 of the anti-B7-H3 antibody are represented by SEQ ID NOs: 4 to 6, respectively.
12. The ligand-drug conjugate or the pharmaceutically acceptable salt or solvate thereof according to embodiment 11, wherein a heavy chain variable region HCVR of the anti-B7-H3 antibody is represented by SEQ ID NO: 7, and a light chain variable region LCVR of the anti-B7-H3 antibody is represented by SEQ ID NO: 8.
13. The ligand-drug conjugate or the pharmaceutically acceptable salt or solvate thereof according to embodiment 12, wherein a heavy chain constant region of the anti-B7-H3 antibody is represented by SEQ ID NO: 9 or 10, and a light chain constant region of the anti-B7-H3 antibody is represented by SEQ ID NO: 11.
14. The ligand-drug conjugate or the pharmaceutically acceptable salt or solvate thereof according to any one of embodiments 2, 5 to 7, and 9 to 13, wherein the ligand-drug conjugate has a structure as shown below: 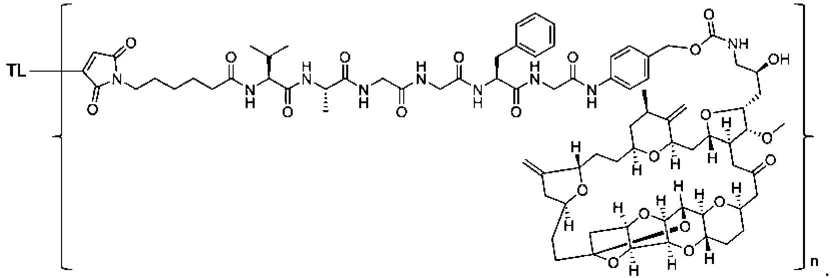
15. A method for preparing the ligand-drug conjugate or the pharmaceutically acceptable salt or solvate thereof according to any one of embodiments 2 to 14, comprising:
   after reduction of the targeting ligand, subjecting the targeting ligand to a conjugation reaction with pre-synthesized -L-D to obtain a compound represented by a general formula of TL-L-D.
16. A pharmaceutical composition, comprising the ligand-drug conjugate or the pharmaceutically acceptable salt or solvate thereof according to any one of embodiments 2 to 14, and a pharmaceutically acceptable excipient, diluent, or carrier.
17. Use of the ligand-drug conjugate or the pharmaceutically acceptable salt or solvate thereof according to any one of embodiments 2 to 14 in preparation of a medicament for treating a tumor.
18. The use according to embodiment 17, wherein the tumor is a solid tumor or hematological tumor such as breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, and leukemia.

## Claims

1. A ligand-drug conjugate of a general formula TL-(L-D)n, or a pharmaceutically acceptable salt or solvate thereof,
wherein TL is a targeting ligand, L is a linker unit, D is Eribulin, and n is a positive integer from 1 to 20;
wherein the ligand-drug conjugate has a structure as shown below: 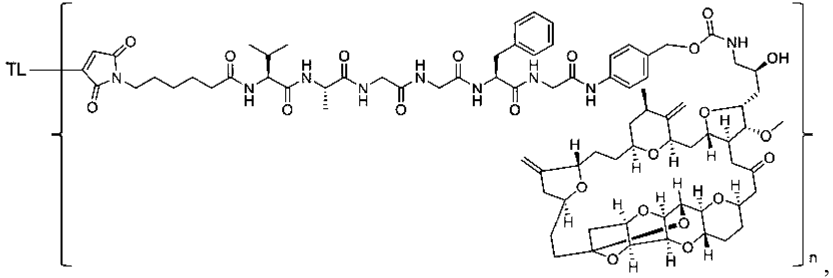
wherein the targeting ligand is an anti-B7-H3 antibody, wherein heavy-chain complementarity-determining regions HCDR1, HCDR2, and HCDR3 of the anti-B7-H3 antibody are represented by SYAVS (SEQ ID NO: 1), SISGGGIYIYYPDSVKG (SEQ ID NO: 2) and HGGAGYFDY (SEQ ID NO: 3) respectively, and light-chain complementarity-determining regions LCDR1, LCDR2, and LCDR3 of the anti-B7-H3 antibody are represented by RGSESVHSYLA (SEQ ID NO: 4), NAKTLQS SEQ ID NO: 5 and QQHYGSPPWT (SEQ ID NO: 6) respectively.

2. The ligand-drug conjugate or the pharmaceutically acceptable salt or solvate thereof according to claim 1, wherein a heavy chain variable region HCVR of the anti-B7 H3 antibody is represented by SEQ ID NO: 7, and a light-chain variable region LCVR of the anti-B7 H3 antibody is represented by SEQ ID NO: 8.

3. The ligand-drug conjugate or the pharmaceutically acceptable salt or solvate thereof according to claim 1 or claim 2, wherein a heavy chain constant region of the anti-B7 H3 antibody is represented by SEQ ID NO: 9 or 10, and a light-chain constant region of the anti-B7 H3 antibody is represented by SEQ ID NO: 11.

4. The ligand-drug conjugate or the pharmaceutically acceptable salt or solvate thereof according to claim 3, wherein the heavy-chain constant region of the anti-B7 H3 antibody is represented by SEQ ID NO: 9.

5. The ligand-drug conjugate or the pharmaceutically acceptable salt or solvate thereof according to any one of claims 1 to 4, wherein n = 1 to 8.

6. The ligand-drug conjugate or the pharmaceutically acceptable salt or solvate thereof according to any one of claims 1 to 5, wherein n = 4 to 8.

7. A method for preparing the ligand-drug conjugate or the pharmaceutically acceptable salt or solvate thereof according to any one of claims 1 to 6, comprising:
after reduction of the targeting ligand, subjecting the targeting ligand to a conjugation reaction with pre-synthesized -L-D to obtain the compound represented by the general formula of TL-(L-D)n.

8. A pharmaceutical composition, comprising the ligand-drug conjugate or the pharmaceutically acceptable salt or solvate thereof according to any one of claims 1 to 6, and a pharmaceutically acceptable excipient, diluent, or carrier.

9. The ligand-drug conjugate or the pharmaceutically acceptable salt or solvate thereof according to any one of claims 1 to 6 for use as a medicament.

10. The ligand-drug conjugate or the pharmaceutically acceptable salt or solvate thereof according to any one of claims 1 to 6 for use in treating a tumor.

11. The use according to claim 10, wherein the tumor is a solid tumor or hematological tumor, such as breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma multiforme, sarcoma, lymphoma, or leukemia.
